# EUROPEAN PATENT SPECIFICATION

(11) **EP 3 250 676 B1**
(45) Date of publication and mention of the grant of the patent: **11.08.2021**
(21) Application number: 15879399.2
(22) Date of filing: 30.01.2015
(51) Int. Cl.: C12N 1/20, C12Q 1/04, C12Q 1/6886, C12Q 1/689

(54) **BIOMARKERS FOR COLORECTAL CANCER RELATED DISEASES**
BIOMARKER FÜR ERKRANKUNGEN IM ZUSAMMENHANG MIT KOLOREKTALKREBS
BIOMARQUEURS POUR MALADIES LIÉES À UN CANCER COLORECTAL

(43) Date of publication of application: 06.12.2017
(73) Proprietor: BGI Shenzhen, Shenzhen, Guangdong 518083 (CN)
(72) Inventor: FENG, Qiang, Shenzhen Guangdong 518083 (CN); LIANG, Suisha, Shenzhen Guangdong 518083 (CN); JIA, Huijue, Shenzhen Guangdong 518083 (CN); WANG, Jun, Shenzhen Guangdong 518083 (CN)
(74) Representative: Lavoix
(86) International application number: PCT/CN2015/071896
(87) International publication number: WO 2016/119191

(56) References cited:
- WO-A1-2014/019271
- WO-A1-2014/019408
- WO-A2-02/101357
- WO-A2-2005/083128
- US-A1- 2014 107 092
- IRADJ SOBHANI ET AL: "Microbial dysbiosis in colorectal cancer (CRC) patients.", PLOS ONE 27 JAN 2011, vol. 6, no. 1, 27 January 2011 (2011-01-27), page e16393, XP002650756, ISSN: 1932-6203
- WEIGUANG CHEN ET AL: "Human intestinal lumen and mucosa-associated microbiota in patients with colorectal cancer", PLOS ONE, PUBLIC LIBRARY OF SCIENCE, US, [Online] vol. 7, no. 6, June 2012 (2012-06), pages 1-9, XP003031297, ISSN: 1932-6203, DOI: 10.1371/JOURNAL.PONE.0039743 [retrieved on 2012-06-28]
- JULIAN R. MARCHESI ET AL: "Towards the Human Colorectal Cancer Microbiome", PLOS ONE, vol. 6, no. 5, 24 May 2011 (2011-05-24), page e20447, XP55019542, DOI: 10.1371/journal.pone.0020447
- J. AHN ET AL: "Human Gut Microbiome and Risk for Colorectal Cancer", JNCI JOURNAL OF THE NATIONAL CANCER INSTITUTE, vol. 105, no. 24, 6 December 2013 (2013-12-06), pages 1907-1911, XP055133612, ISSN: 0027-8874, DOI: 10.1093/jnci/djt300
- TINGTING WANG ET AL: "Structural segregation of gut microbiota between colorectal cancer patients and healthy volunteers", THE ISME JOURNAL, vol. 6, no. 2, 18 August 2011 (2011-08-18) , pages 320-329, XP55152252, ISSN: 1751-7362, DOI: 10.1038/ismej.2011.109
- TIFFANY L. WEIR ET AL: "Stool Microbiome and Metabolome Differences between Colorectal Cancer Patients and Healthy Adults", PLOS ONE, vol. 8, no. 8, 6 August 2013 (2013-08-06), page e70803, XP055214589, DOI: 10.1371/journal.pone.0070803
- WEI-LIN WANG ET AL: "Application of metagenomics in the human gut microbiome", WORLD JOURNAL OF GASTROENTEROLOGY, vol. 21, no. 3, 21 January 2015 (2015-01-21), page 803, XP055475301, CN ISSN: 1007-9327, DOI: 10.3748/wjg.v21.i3.803
- JOSE U SCHER ET AL: "Expansion of intestinal Prevotella copri correlates with enhanced susceptibility to arthritis", ELIFE, vol. 2, 5 November 2013 (2013-11-05), XP055475267, DOI: 10.7554/eLife.01202
- QIANG FENG ET AL: "Gut microbiome development along the colorectal adenoma-carcinoma sequence", NATURE COMMUNICATIONS, vol. 6, 11 March 2015 (2015-03-11), page 6528, XP055333614, DOI: 10.1038/ncomms7528
- QIN, JUNJIE ET AL.: 'A metagenome-wide association study of gut microbiota in type 2 diabetes.' NATURE vol. 490, no. 7418, 26 September 2012, pages 55 - 60, XP055111695 DOI: 10.1038/NATURE11450
- QIN, JUNJIE ET AL.: 'A human gut microbial gene catalogue established by metagenomic sequencing.' NATURE vol. 464, no. 7285, 04 March 2010, pages 59 - 65, XP008132800 DOI: 10.1038/NATURE08821
- None

## Description

### FIELD

The present invention relates to biomarkers and methods for predicting the risk of colorectal cancer.

### BACKGROUND

Colorectal cancer (CRC) is among the top three most frequently diagnosed cancer worldwide and a leading cause of cancer mortality. The incidence is higher in more developed countries, but is rapidly increasing in historically low risk areas such as Eastern Asia, Spain and Eastern Europe, attributable to a so-called western lifestyle. Genetic changes accumulate for many years in the development of colorectal cancer, often involving loss of the tumour suppressor gene adenomatous polyposis coli (*APC*)*,* followed by activating and inactivating mutations in *KRAS, PIK3CA* and *TP53,* respectively (Brenner, H., Kloor, M. & Pox, C. P. Colorectal cancer. Lancet383, 1490-502 (2014)). Most CRC cases are sporadic, but are preceded by dysplastic adenomas which could progress into malignant forms, referred to as the adenoma-carcinoma sequence. Early diagnosis of colorectal adenoma and carcinoma will help to not only prevent mortality, but also reduce the costs for surgical intervention.

CRC is among the most studied diseases implicated with the gut microbiota. Causal relationships, however, were typically investigated by application of antibiotic cocktails that eradicate the gut microbiota without knowing the exact microbial strains and genes at play. *Fusobacterium* has been detected in colorectal carcinoma relative to normal colon tissue, and was found to be enriched in adenomas. *Fusobacterium nucleatum,* a periodontal pathogen, has been shown to promote myeloid infiltration of intestinal tumours in *Apc*^{*Min*/+} mice and associate with increased expression of proinflammatory genes such as *Ptgs2*(*COX-2*)*, Scyb1* (*IL8*), *Il6, Tnf*(*TNFα*), and *Mmp3* in mice and humans (Kostic, A. D. et al. Fusobacterium nucleatum potentiates intestinal tumorigenesis and modulates the tumor-immune microenvironment. Cell Host Microbe14, 207-215 (2013)). It is not clear, however, whether more bacteria or archaea serve as markers for, or contribute to the aetiology of colorectal carcinomas.

Current tests of CRC, such as flexible sigmoidoscopy and colonoscopy, are invasive and patients may find the procedures and bowel preparation to be uncomfortable or unpleasant. Interactions between the gut microbiota and the immune system have an important role in many diseases both within and outside the gut (Cho, I. &Blaser, M. J. The human microbiome: at the interface of health and disease. Nature Rev. Genet. 13, 260-270 (2012)).

Intestinal microbiota analysis of feces DNA has the potential to be used as a non-invasive test for finding specific biomarkers that may be used as a screening tool for early diagnosis of patients having CRC, thus leading to a longer survival and a better quality of life.

### SUMMARY

Embodiments of the present disclosure seek to solve at least one of the problems existing in the prior art to at least some extent.

The present invention is defined by the scope of the appended claims and is based on the following findings by the inventors:
Assessment and characterization of gut microbiota has become a major research area in human disease, including colorectal cancer. The inventors presented for the first time the metagenomic whole-genome shotgun sequencing for faecal samples from healthy controls, colorectal carcinoma and adenoma patients. To carry out analysis on gut microbial content in colorectal carcinoma and adenoma patients, the inventors carried out a protocol for a Metagenome-Wide Association Study (MGWAS) (Qin, J. et al. A metagenome-wide association study of gut microbiota in type 2 diabetes. Nature 490, 55-60 (2012)).

For comparison of the faecal microbiome in healthy controls, advanced adenoma and carcinoma patients, genes that showed significant difference in relative abundance between any of the two groups were identified (p< 0.05, Kruskal-Wallis test). These marker genes were then clustered into MLGs (metagenomic linkage groups) (Qin et al. 2012, *supra*) according to their abundance variation across all samples, and the inventors identified MLG characteristic of the tumours. Then the inventors identified and validated 15 MLGs for early and non-invasive diagnosis of colorectal carcinoma, and 10 MLGs for early and non-invasive diagnosis of colorectal adenoma. To exploit the potential ability of these CRC classification by gut microbiota, the inventors calculated probability of illness througha random forest model based on the 15 MLGs and 10 MLGs respectively. The inventors' data provide insight into the characteristics of the gut metagenome related to CRC risk, a paradigm for future studies of the pathophysiological role of the gut metagenome in other relevant disorders, and the potential usefulness for a gut-microbiota-based approach for assessment of individuals at risk of such disorders.

It is believed that the 15 MLGs and 10 MLGs are valuable for increasing CRC detection at earlier stages due to the following. First, the markers of the present invention are more specific and sensitive as compared with conventional markers. Second, analysis of stool promises accuracy, safety, affordability, and patient compliance. And samples of stool are transportable. As compared with colonoscopy requiring bowel preparation, the present invention relates to an *in vitro* method, which is comfortable and noninvasive, so people will participate in a given screening program more easily. Third, the markers of the present invention may also serve as tools for therapy monitoring in CRC patients to detect the response to therapy.

Therefore, in a first aspect, the present invention provides a nucleic acid biomarker set for predicting or diagnosing a disease related to microbiota in a subject or determining whether a subject is at the risk of developing the disease, wherein the disease is colorectal cancer.

In a second aspect, the present invention provides use of a kit for predicting or diagnosing a disease related to microbiota in a subject or determining whether a subject is at the risk of developing the disease, comprising agents for determining the level or amount of each biomarker of the biomarker set of the present invention in a sample, wherein the disease is colorectal cancer.

In a third aspect, the present invention provides a method for predicting or diagnosing a disease related to microbiota in a subject or determining whether a subject is at the risk of developing the disease, wherein the disease is colorectal cancer.

### BRIEF DISCRIPTION OF DRAWINGS

These and other aspects and advantages of the present disclosure will become apparent and more readily appreciated from the following descriptions taken in conjunction with the drawings, in which:

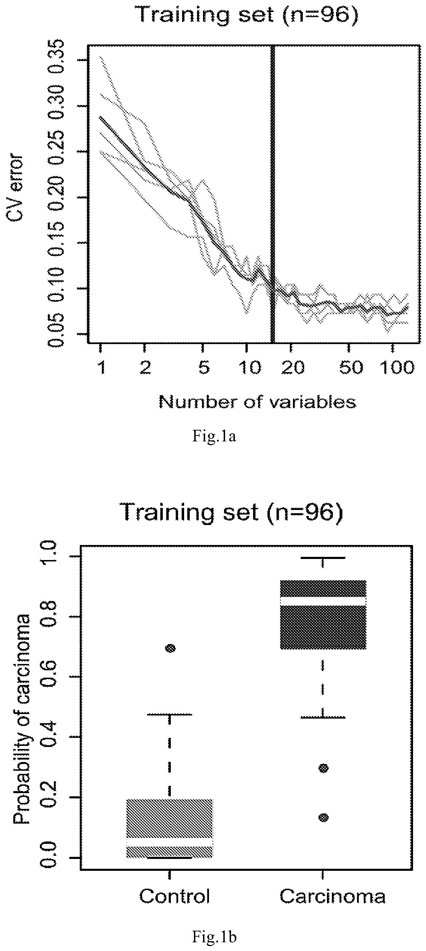

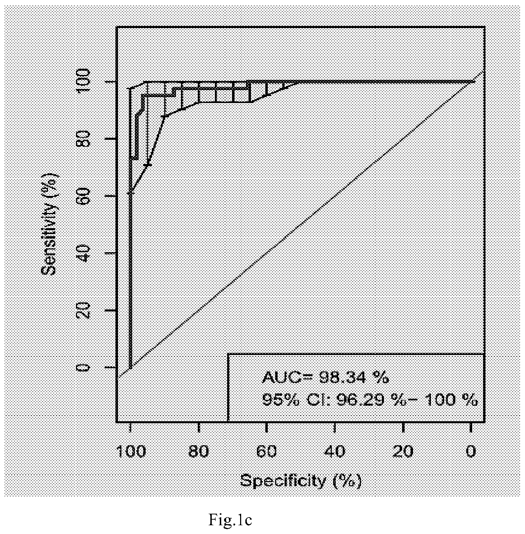

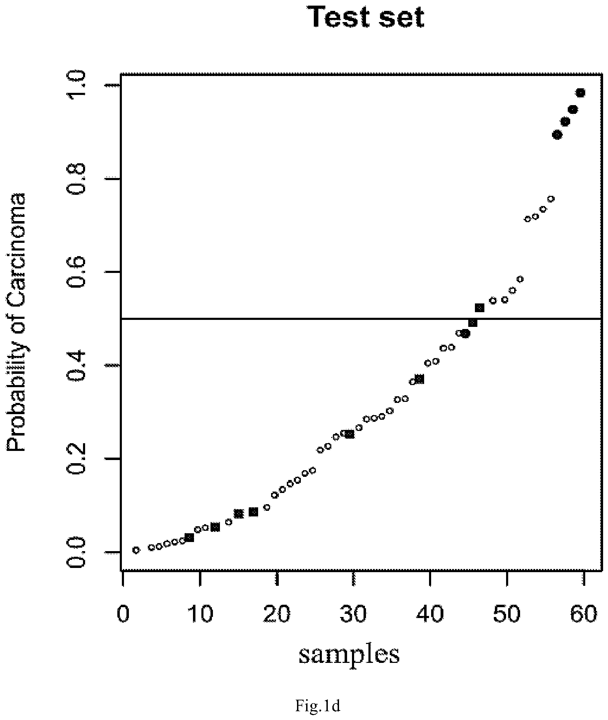

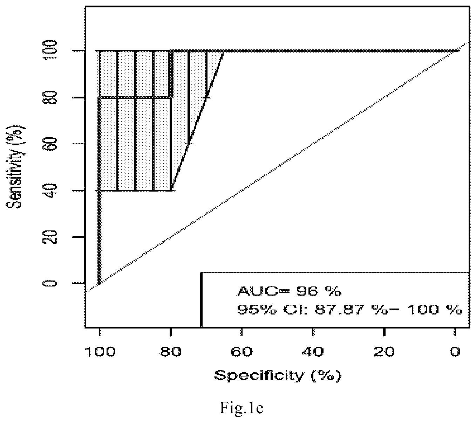

**Fig.1** **shows that gut MLGs classify colorectal carcinoma samples from healthy controls. (a)** Distribution of 5 trials of 10-fold cross-validation error in random forest classification of carcinoma as the number of MLGs increases. The model was trained using relative abundance of the MLGs (> 100 genes) in the controls and carcinoma samples (n = 55 and 41). The black curve indicates average of the 5 trials (grey lines). The black line marks the number of MLGs (15 MLGs) in the optimal set (Table 2-1, Table 2-2, Table 3). The same MLGs were selected if age and BMI were included along with the MLGs. **(b)** Box-and-whisker plot for the probability of carcinoma in the cross-validational training set according to the model in **(a). (c)** Receiver operating curve (ROC) for the training set. At the cut-off 0.5, the AUC is 98.34%, 95% confidence interval (CI) is 96.29-100%. **(d)** Classification of the test set consisted of 8 controls (black square), 47 advanced adenoma (hollow circle) and 5 carcinoma (solid black circle). **(e)** ROC for the test set. At the cut-off 0.5, the AUC is 96%, 95% CI is 87.88-100%. If the probability of carcinoma≥0.5, the subject is at risk of colorectal carcinoma. The results of Fig.1 demonstrated that the 15 MLGs could be used as biomarkers for diagnosis of colorectal carcinoma and/or determining the risk of colorectal carcinoma with high sensitivity and high specificity.

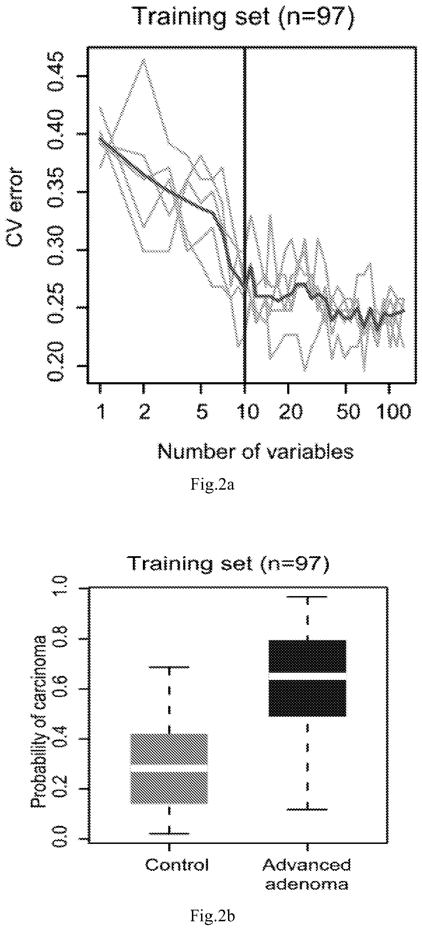

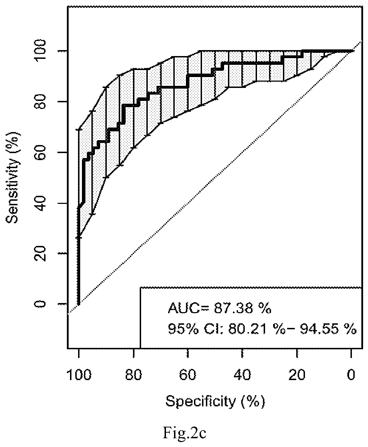

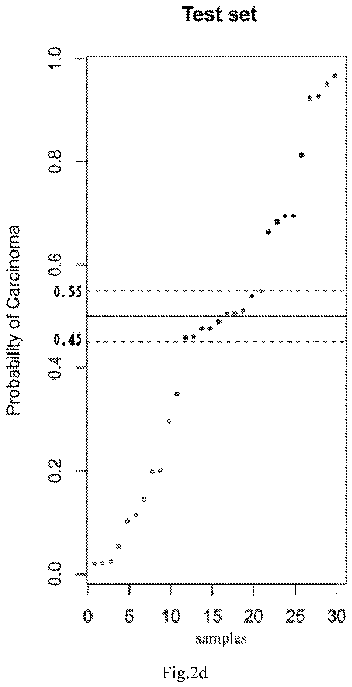

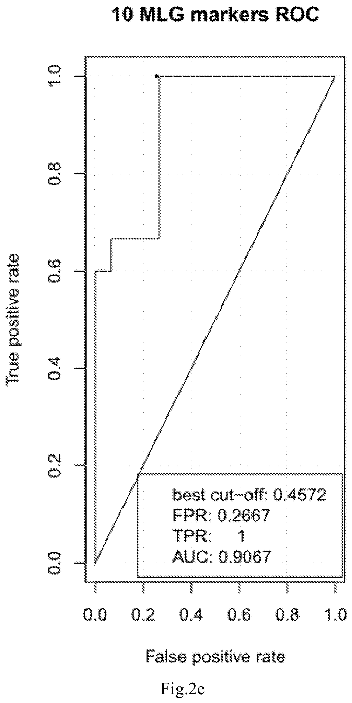

**Fig.2** **shows that gut MLGs classify colorectal adenoma samples from healthy controls. (a)** Distribution of 5 trials of 10-fold cross-validation error in random forest classification of advanced adenoma as the number of MLGs increases. The model was trained using relative abundance of the MLGs (> 100 genes) in the controls and advanced adenoma samples (n = 55 and 42). The black curve indicates average of the 5 trials (grey lines). The black line marks the number of MLGs (10 MLGs) in the optimal set (Table 7-1, Table 7-2). The same MLGs were selected if age and BMI were included along with the MLGs. **(b)** Box-and-whisker plot for the probability of advanced adenoma in the cross-validational training set according to the model in **(a). (c)** Receiver operating curve (ROC) for the training set. At the cut-off 0.5, the AUC is 87.38%, 95% confidence interval (CI) is 80.21-94.55%. **(d)** Classification of the test set consisted of 15 controls (hollow circle) and 15 advanced adenoma (solid black circle). **(e)** ROC for the test set. At the best cut-off 0.4572, the AUC is 90.67%, true positive rate (TPR) is 1, and false positive rate (FPR) is 0.2667. If the probability of colorectal adenoma ≥ 0.4572 (the best cut-off), the subject is at risk of colorectal adenoma. The results of Fig.2 demonstrated that the 10 MLGs could be used as biomarkers for diagnosis of advanced adenoma and/or determining the risk of advanced adenoma with high sensitivity and high specificity.

### DETAILED DESCRIPTION

Terms used herein have meanings as commonly understood by a person of ordinary skill in the fields to which the present invention is relevant. However, in order to better understand the invention, the definitions and explanations of the relevant terms are provided as follows.

Terms such as "a", "an" and "the" are not intended to refer to only a singular entity, but include the general class of which a specific example may be used for illustration.

According to the invention, the term "a biomarker", also named "a biological marker", refers to a measurable indicator of a biological state or condition of a subject. Such biomarkers can be any substance in the subject, for example, nucleic acid marker (e.g. DNA), protein marker, cytokine marker, chemokine marker, carbohydrate marker, antigen marker, antibody marker, species marker (species/genus marker) and functions marker (KO/OG marker) and the like, provided that they are in relation with a particular biological state or condition (such as, a disease) of the subject. Biomarkers are often measured and evaluated to examine normal biological processes, pathogenic processes, or pharmacologic responses to a therapeutic intervention, and are useful in many scientific fields.

According to the invention, the term "a biomarker set" refers to a set of biomarkers (that is, a combination of two or more biomarkers).

According to the invention, the term "a disease related to microbiota" refers to a disease that is in relation with imbalance of microbiota in gut. For example, the disease may be caused, induced or aggravated by imbalance of microbiota in gut. Such a disease may be advanced adenoma of colorectum or colorectal cancer/carcinoma.

According to the invention, the term "a subject" refers to an animal, particularly a mammal, such as a primate, preferably human.

According to the invention, the expression "colorectal cancer" has the same meaning as "colorectal carcinoma".

According to the invention, the expression "advanced adenoma" and "colorectal advanced adenoma" have the same meaning as "advanced adenoma in colorectum".

According to the invention, the expression "cutoff" and "cut-off' have the same meaning, referring to a predicted critical value. The predicted critical value can be obtained by conventional experiments, for example by determining relative abundances of biomarkers in the samples from subjects with known physiological status through parallel testings.

According to the invention, the term "MLG" is defined as a group of genetic material in a metagenome that is probably physically linked as a unit rather than being independently distributed (See, Qin, J. et al. A metagenome-wide association study of gut microbiota in type 2 diabetes. Nature 490, 55-60 (2012)). It allows to avoid the need to completely determine the specific microbial species present in the metagenome, which is important given there are a large number of unknown organisms and that there is frequent lateral gene transfer (LGT) between bacteria. In the present invention, MLG refers to a group of genes that has a consistent abundance level and taxonomic assignment.

According to the invention, the term "a specific fragment" of a MLG refers to a frament of MLG which is unique for the MLG. Conventional methods can be used for determine whether a fragment is unique for the MLG which it is derived from. For example, one may input the sequence of the fragment into a public database (such as GenBank) and carry out BLAST programme. If the fragment is only present in one species in the database (in this case, the MLG would represent or correspond to the species) or if there is no homolog with at least 90% identity (such as 95% identity) with the fragment in the database (in this case, the MLG would refer to an unknown species), the fragment may be considered as unique. As discussed above, a MLG generally refers to a specific microbial species (either known or unknown), and thus "a specific fragment" of the MLG may also be considered as a genomic fragment unique for the specific microbial species (that is, the fragment is only present in the specific microbial species).

According to the invention, the term "identity" refers to the match degree between two polypeptides or between two nucleic acids. When two sequences for comparison have the same base or amino acid monomer sub-unit at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison x 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970).

According to the invention, the expression "agents for determining the level or amount of a biomarker" refers to agents that could be used to quantitate or measure the level or amount of a biomarker in a sample. Such agents can be readily designed or obtained by a conventional method well known in the art, on the basis of the sequences for the biomarkers as provided in the present invention. For example, such agents include, but are not limited to, PCR primers that can be used to quantitate or measure the level or amount of a biomarker by e.g. Realtime PCR; probes that can be used to quantitate or measure the level or amount of a biomarker by e.g. a quantitative Southern blot; a microarray that can be used to quantitate or measure the level or amount of a biomarker, e.g. a gene chip; and the like. In addition, as known in the art, the second generation sequencing method or the third generation sequencing method may also be used to quantitate or measure the level or amount of a biomarker. Therefore, such agents can also be agents for carrying out the second generation sequencing method or the third generation sequencing method, which are commercially available.

According to the invention, the expression "primers which are capable of specifically amplifying" a certain nucleic acid or a certain sequence means that the primers, when used for amplification (e.g. PCR amplification), specifically anneal to the certain nucleic acid or sequence, and produce a unique amplification product (that is, do not anneal to other nucleic acids or sequences or produce other side products).

According to the invention, the expression "probes which are capable of specifically hybridizing with" a certain nucleic acid or a certain sequence means that the probes, when used for hybridization or detection under a strigency condition, specifically anneal to and hybridize with the certain nucleic acid or sequence, but do not anneal to or hybridize with other nucleic acids or sequences.

It is common knowledge for a person skilled in the art to design said primers or probes, based on a particular sequence (such as a particular MLG or a specific fragment thereof). For example, such common knowledge may be found in a variety of textbooks (see, for example, J. Sambrook et al., Molecular Cloning: Laboratory Manual, the second edition, Cold Spring Harbor Laboratory Press, 1989; F. M. Ausubel et al., Short Protocols in Molecular Biology, the third edition, John Wiley & Sons, Inc.; and a lot of papers, such as Buck et al. (1999), Lowe et al. (1990) and so on.

According to the invention, the term "the second generation sequencing method" refers to a new generation DNA sequencing method, which is developed in current years and represented by Illumina GA, Roche 454, ABI Solid, and the like; and is distinguished from the traditional sequencing method (such as, Sanger sequencing method). The difference between the second generation sequencing method and the traditional sequencing method (such as, Sanger sequencing method) lies in that the second generation sequencing method analyses a DNA sequence by conducting sequencing as carrying out synthesis. The second generation sequencing method has the following advantages: 1) low cost, which is 1% of the cost of the traditional sequencing method; 2) high throughput, capable of conducting sequencing to multiple samples simultaneously, and capable of producing a data of about 50 billion (50G) bases for one Solexa sequencing; 3) high accuracy (greater than 98.4%), which effectively solve the problem relating to readout of poly-repetitive sequences. In another aspect, when the number of the sequences to be sequenced is predetermined, the high sequencing throughput in turn improves the sequencing depth of the sequences (for example, each sequence can be sequenced for more times), thereby ensuring the credibility of the sequencing result.

According to the invention, the term "the third generation sequencing method" refers to a new generation of single-molecule sequencing technology which is developed recently. The third generation of sequencing technologies offer advantages over current sequencing technologies, including (i) higher throughput; (ii) faster turnaround time (e.g. sequencing metazoan genomes at high fold coverage in minutes); (iii) longer read lengths to enhance *de novo* assembly and enable direct detection of haplotypes and even whole chromosome phasing; (iv) higher consensus accuracy to enable rare variant detection; (v) small amounts of starting material (theoretically only a single molecule may be required for sequencing); and (vi) low cost, where sequencing the human genome at high fold coverage for less than $100 is now a reasonable goal for the community. For further details about the third generation sequencing method, see, e.g. Eric E. Schadt et al., A window into third-generation sequencing, Human Molecular Genetics, 2010, Vol.19, Review Issue 2, R227-R240.

According to the invention, the term "relative abundance" has the common meaning as known in the art, and can be calculated by a method known in the art. For example, the relative abundance of a gene (i.e. a biomarker) or a MLG can be determined or calculated by the method as disclosed in Qin, J. et al. A metagenome-wide association study of gut microbiota in type 2 diabetes. Nature 490, 55-60 (2012).

It would be appreciated by those skilled in the art that the terminology herein is provided for better understanding of the present invention claims.

In a first aspect, the present invention provides a nucleic acid biomarker set for predicting or diagnosing a disease related to microbiota, wherein the disease is colorectal carcinoma, in a subject or determining whether a subject is at the risk of developing the disease, comprising the following biomarkers (all the biomarkers were listed in Table 3):
(1) MLG 5045 consisting of nucleic acids with the sequence of SEQ ID NO: 3732-3918; and
(2) MLG 121 consisting of nucleic acids with the sequence of SEQ ID NO: 3919-6548 optionally, the biomarker set further comprises one or more of the following biomarkers:
(3) MLG 75 consisting of nucleic acids with the sequence of SEQ ID NO: 1350-1527;
(4) MLG 109 consisting of nucleic acids with the sequence of SEQ ID NO: 6549-7235;
(5) MLG 317 consisting of nucleic acids with the sequence of SEQ ID NO: 7581-7700;
(6) MLG 135 consisting of nucleic acids with the sequence of SEQ ID NO: 2230-3731;
(7) MLG 223 consisting of nucleic acids with the sequence of SEQ ID NO: 9892-11298;
(8) MLG 100 consisting of nucleic acids with the sequence of SEQ ID NO: 9596-9891;
(9) MLG 219 consisting of nucleic acids with the sequence of SEQ ID NO: 7701-8028;
(10) MLG 114 consisting of nucleic acids with the sequence of SEQ ID NO: 1528-2089;
(11) MLG 84 consisting of nucleic acids with the sequence of SEQ ID NO: 1-165;
(12) MLG 166 consisting of nucleic acids with the sequence of SEQ ID NO: 7236-7580;
(13) MLG 2985 consisting of nucleic acids with the sequence of SEQ ID NO: 8029-9595;
(14) MLG 131 consisting of nucleic acids with the sequence of SEQ ID NO: 166-1349; and
(15) MLG 1564 consisting of nucleic acids with the sequence of SEQ ID NO: 2090-2229.

In a preferable embodiment, the nucleic acid biomarker set of the invention comprises the biomarkers as defined in (1)-(13).

As known by a skilled person in the art, a specific fragment can be of any length, provided that such a fragment is unique for the MLG which it is derived from or a species represented by the MLG (that is, the fragment does not exist in other MLGs or other species). However, for the convenience, the specific fragment may be at least 30 bp, or at least 40 bp, or at least 50 bp, or at least 60 bp, or at least 70 bp, or at least 80 bp, or at least 90 bp, or at least 100 bp, or at least 150 bp, or at least 200 bp, or at least 250 bp, or at least 300 bp, or at least 350 bp, or at least 400 bp, or at least 450 bp, or at least 500 bp, or at least 600 bp, or at least 700 bp, or at least 800 bp, or at least 900 bp, or at least 1000 bp, or at least 1500 bp, or at least 2000 bp, in length.

In an example, which is not part of the invention, the nucleic acid biomarker set of the invention may be further characterized by any one or more of the following items:
(1) one or more specific fragments of MLG 84 are selected from the group consisting of SEQ ID NO: 1-165 or any combination thereof;
(2) one or more specific fragments of MLG 131 are selected from the group consisting of SEQ ID NO: 166-1349 or any combination thereof;
(3) one or more specific fragments of MLG 75 are selected from the group consisting of SEQ ID NO: 1350-1527 or any combination thereof;
(4) one or more specific fragments of MLG 114 are selected from the group consisting of SEQ ID NO: 1528-2089 or any combination thereof;
(5) one or more specific fragments of MLG 1564 are selected from the group consisting of SEQ ID NO: 2090-2229 or any combination thereof;
(6) one or more specific fragments of MLG 135 are selected from the group consisting of SEQ ID NO: 2230-3731 or any combination thereof;
(7) one or more specific fragments of MLG 5045 are selected from the group consisting of SEQ ID NO: 3732-3918 or any combination thereof;
(8) one or more specific fragments of MLG 121 are selected from the group consisting of SEQ ID NO: 3919-6548 or any combination thereof;
(9) one or more specific fragments of MLG 109 are selected from the group consisting of SEQ ID NO: 6549-7235 or any combination thereof;
(10) one or more specific fragments of MLG 166 are selected from the group consisting of SEQ ID NO: 7236-7580 or any combination thereof;
(11) one or more specific fragments of MLG 317 are selected from the group consisting of SEQ ID NO: 7581-7700 or any combination thereof;
(12) one or more specific fragments of MLG 219 are selected from the group consisting of SEQ ID NO: 7701-8028 or any combination thereof;
(13) one or more specific fragments of MLG 2985 are selected from the group consisting of SEQ ID NO: 8029-9595 or any combination thereof;
(14) one or more specific fragments of MLG 100 are selected from the group consisting of SEQ ID NO: 9596-9891 or any combination thereof; and
(15) one or more specific fragments of MLG 223 are selected from the group consisting of SEQ ID NO: 9892-11298 or any combination thereof.

In a preferable embodiment, the subject is a mammal, such as a primate, preferably human.

In a preferable embodiment, the biomarker set of the present invention is used for distinguishing a patient with colorectal carcinoma from a healthy subject and a patient with advanced adenoma.

In a second aspect, the present invention provides the use of a kit in the method of the present invention for predicting or diagnosing a disease related to microbiota in a subject or determining whether a subject is at the risk of developing the disease, wherein the disease is colorectal carcinoma, comprising agents for determining the level or amount of each biomarker of the nucleic acid biomarker set according to the invention in a sample, wherein the agents are selected from the group consisting of:
(a) a primer set, comprising:
   (a1) one or more primers which are capable of specifically amplifying MLG 5045 consisting of SEQ ID NO: 3732-3918 or one or more specific fragments thereof; and
   (a2) one or more primers which are capable of specifically amplifying MLG 121 consisting of SEQ ID NO: 3919-6548;
      optionally, the primer set further comprises one or more of the following primers:
      (a3) one or more primers which are capable of specifically amplifying MLG 75 consisting of SEQ ID NO: 1350-1527;
      (a4) one or more primers which are capable of specifically amplifying MLG 109 consisting of SEQ ID NO: 6549-7235;
      (a5) one or more primers which are capable of specifically amplifying MLG 317 consisting of SEQ ID NO: 7581-7700;
      (a6) one or more primers which are capable of specifically amplifying MLG 135 consisting of SEQ ID NO: 2230-3731;
      (a7) one or more primers which are capable of specifically amplifying MLG 223 consisting of SEQ ID NO: 9892-11298;
      (a8) one or more primers which are capable of specifically amplifying MLG 100 consisting of SEQ ID NO: 9596-9891;
      (a9) one or more primers which are capable of specifically amplifying MLG 219 consisting of SEQ ID NO: 7701-8028;
      (a10) one or more primers which are capable of specifically amplifying MLG 114 consisting of SEQ ID NO: 1528-2089;
      (a11) one or more primers which are capable of specifically amplifying MLG 84 consisting of SEQ ID NO: 1-165;
      (a12) one or more primers which are capable of specifically amplifying MLG 166 consisting of SEQ ID NO: 7236-7580;
      (a13) one or more primers which are capable of specifically amplifying MLG 2985 consisting of SEQ ID NO: 8029-9595;
      (a14) one or more primers which are capable of specifically amplifying MLG 131 consisting of SEQ ID NO: 166-1349; and
      (a15) one or more primers which are capable of specifically amplifying MLG 1564 consisting of SEQ ID NO: 2090-2229;
(b) a probe set, comprising:
   (b1) one or more probes which are capable of specifically hybridizing with MLG 5045 consisting of SEQ ID NO: 3732-3918; and
   (b2) one or more probes which are capable of specifically hybridizing with MLG 121 consisting of SEQ ID NO: 3919-6548;
      optionally, the probe set further comprises one or more of the following probes:
      (b3) one or more probes which are capable of specifically hybridizing with MLG 75 consisting of SEQ ID NO: 1350-1527;
      (b4) one or more probes which are capable of specifically hybridizing with MLG 109 consisting of SEQ ID NO: 6549-7235;
      (b5) one or more probes which are capable of specifically hybridizing with MLG 317 consisting of SEQ ID NO: 7581-7700;
      (b6) one or more probes which are capable of specifically hybridizing with MLG 135 consisting of SEQ ID NO: 2230-3731;
      (b7) one or more probes which are capable of specifically hybridizing with MLG 223 consisting of SEQ ID NO: 9892-11298;
      (b8) one or more probes which are capable of specifically hybridizing with MLG 100 consisting of SEQ ID NO: 9596-9891;
      (b9) one or more probes which are capable of specifically hybridizing with MLG 219 consisting of SEQ ID NO: 7701-8028;
      (b10) one or more probes which are capable of specifically hybridizing with MLG 114 consisting of SEQ ID NO: 1528-2089;
      (b11) one or more probes which are capable of specifically hybridizing with MLG 84 consisting of SEQ ID NO: 1-165;
      (b12) one or more probes which are capable of specifically hybridizing with MLG 166 consisting of SEQ ID NO: 7236-7580;
      (b13) one or more probes which are capable of specifically hybridizing with MLG 2985 consisting of SEQ ID NO: 8029-9595;
      (b14) one or more probes which are capable of specifically hybridizing with MLG 131 consisting of SEQ ID NO: 166-1349 o; and
      (b15) one or more probes which are capable of specifically hybridizing with MLG 1564 consisting of SEQ ID NO: 2090-2229;
(c) a microarray comprising the primer set of (a) and/or the probe set of (b); and
(d) any combination of (a)-(c).

In a preferable embodiment, the primer set comprises the primers as defined in (a1)-(a13).

In a preferable embodiment, the probe set comprises the probes as defined in (b1)-(b13).

In a preferable embodiment, the kit is used for predicting or diagnosing a disease related to microbiota in a subject or determining whether a subject is at the risk of developing the disease via a method comprising the following steps:
(1) determining the level or amount of each biomarker of the biomarker set according to the invention in a sample from the subject by using the kit; and
(2a) calculating a probability of the disease by comparing the level or amount of each biomarker in the sample with a training dataset using a Multivariate statistical model (such as a random Forest model);
   wherein the probability of the disease greater than a cutoff indicates that the subject has or is at the risk of developing the disease; or
(2b) comparing the level or amount of each biomarker in the sample with the level or amount of the corresponding biomaker in the control; preferably, the control is a plurality of healthy subjects;
wherein as compared with the control, the increase of the level or amount of the biomarkers in the sample indicates that the subject has or is at the risk of developing the disease.

In a preferable embodiment, the training dataset comprises the data about the level or amount of each biomarker of a plurality of subjects having the disease and a plurality of healthy subjects.

In a preferable embodiment, the training dataset comprises the data in Tables 4-1 and 4-2, and a probability greater than a cutoff of 0.5 indicates that the subject has or is at the risk of developing the disease.

In a preferable embodiment, the subject is a mammal, such as a primate, preferably human.

In a preferable embodiment, the sample is a fecal sample.

In a preferable embodiment, the level or amount of each biomarker is the relative abundance of each biomarker in the sample.

In a preferable embodiment, the kit further comprise addition agents, such as an agent for treating the sample (e.g. sterile water), an agent for carrying out PCR amplification (e.g. polymerase, dNTP, and amplification buffer), and an agent for carrying out hybridization (such as labeling buffer, hybridization buffer, and washing buffer).

Further described and not part of the invention is the use of agents for determining the level or amount of each biomarker of the biomarker set according to the present invention in the preparation of a kit for predicting or diagnosing a disease related to microbiota in a subject or determining whether a subject is at the risk of developing the disease.

In an example, the agents for determining the level or amount of each biomarker of the biomarker set are as defined above.

In an example, the kit is used for predicting or diagnosing a disease related to microbiota in a subject or determining whether a subject is at the risk of developing the disease via a method comprising the following steps:
(1) determining the level or amount of each biomarker of the biomarker set according to the present invention in a sample from the subject by using the kit; and
(2a) calculating a probability of the disease by comparing the level or amount of each biomarker in the sample with a training dataset using a Multivariate statistical model (such as a random Forest model);
   wherein the probability of the disease greater than a cutoff indicates that the subject has or is at the risk of developing the disease; or
(2b) comparing the level or amount of each biomarker in the sample with the level or amount of the corresponding biomaker in the control; preferably, the control is a plurality of healthy subjects;
wherein as compared with the control, the increase of the level or amount of the biomarkers in the sample indicates that the subject has or is at the risk of developing the disease.

In an example, the training dataset comprises the data about the level or amount of each biomarker of a plurality of subjects having the disease and a plurality of healthy subjects.

In an example, the training dataset comprises the data in Tables 4-1 and 4-2, and a probability greater than a cutoff of 0.5 indicates that the subject has or is at the risk of developing the disease.

In an example, the subject is a mammal, such as a primate, preferably human.

In an example, the sample is a fecal sample.

In an example, the level or amount of each biomarker is the relative abundance of each biomarker in the sample.

In an example, the disease is colorectal carcinoma.

In an example, the kit further comprise addition agents, such as an agent for treating the sample (e.g. sterile water), an agent for carrying out PCR amplification (e.g. polymerase, dNTP, and amplification buffer), and an agent for carrying out hybridization (such as labeling buffer, hybridization buffer, and washing buffer).

In a third aspect, the present invention provides a method for predicting or diagnosing a disease related to microbiota in a subject or determining whether a subject is at the risk of developing the disease, comprising the following steps:
(1) determining the level or amount of each biomarker of the biomarker set according to the present invention in a sample from the subject; and
(2a) calculating a probability of the disease by comparing the level or amount of each biomarker in the sample with a training dataset using a Multivariate statistical model (such as a random Forest model);
   wherein the probability of the disease greater than a cutoff indicates that the subject has or is at the risk of developing the disease; or
(2b) comparing the level or amount of each biomarker in the sample with the level or amount of the corresponding biomaker in the control; preferably, the control is a plurality of healthy subjects;
wherein as compared with the control, the increase of the level or amount of the biomarkers in the sample indicates that the subject has or is at the risk of developing the disease, wherein the disease is colorectal carcinoma.

In a preferable embodiment, the training dataset comprises the data about the level or amount of each biomarker of a plurality of subjects having the disease and a plurality of healthy subjects

In a preferable embodiment, the training dataset comprises the data in Tables 4-1 and 4-2, and a probability greater than a cutoff of 0.5 indicates that the subject has or is at the risk of developing the disease.

In a preferable embodiment, the kit as defined above or the agents as defined above are used in step (1).

In a preferable embodiment, the subject is a mammal, such as a primate, preferably human.

In a preferable embodiment, the sample is a fecal sample.

In a preferable embodiment, the level or amount of each biomarker is the relative abundance of each biomarker in the sample.

In a preferable embodiment, the method is carried out in vitro.

The present invention is further exemplified in the following non-limiting Examples. Unless otherwise stated, parts and percentages are by weight and degrees are Celsius. The agents as used were all commercially available.

### EXAMPLES

### Example1. Identifying and validating biomarkers for evaluating risk of CRC related diseases

### 1.Sample collection and sequencing

### 1.1 Subjects and patients

The study was conducted both in participants of a health screening program according to national screening recommendations for CRC (Stadlmayr, A. et al. Nonalcoholic fatty liver disease: an independent risk factor for colorectal neoplasia. J Intern Med270, 41-49 (2011)) as well as in patients with suspected CRC undergoing colonoscopy as part of the clinical workup at the Department of Internal Medicine, Oberndorf Hospital (Teaching Hospital of the Paracelsus Medical University Salzburg, Austria) between 2010 and 2012. The study was approved by the local ethics committee (Ethikkommission des Landes Salzburg, approval no. 415-E/1262/2-2010) and informed consent was obtained from all participants.

The laxative Klean-Prep® (containing macrogol 59.0 g, sodium sulphate 5.68 g, sodium bicarbonate 1.68 g, NaCl 1.46 g and potassium chloride 0.74 g; Norgine, Marburg, Germany) was used for bowel preparation before colonoscopy. Colonoscopic findings were classified as tubular adenoma, advanced adenoma (i.e. villous or tubulovillous features, size ≥1 cm or high-grade dysplasia), or carcinoma after a combined analysis of macroscopic and histological results (Bond, J. H. Polyp guideline: diagnosis, treatment, and surveillance for patients with colorectal polypsACG Colorectal Polyp Guideline. Am. J. Gastroenterol.95, 3053-3063 (2000), Winawer SJ & AG., Z. The advanced adenoma as the primary target of screening. Gastrointest Endosc Clin N Am12, 1-9 (2002)).

Lesions were classified by location (i.e. right colon including caecum, ascending colon and transverse colon, left colon ranging from the splenic flexure to the sigmoid, and rectum alone).

Data from 147 Caucasians aged between 45-86 years were included in the initial analysis, including 57 healthy controls (24 females, 33 males), 44 advanced adenoma (22 females, 22 males) and 46 carcinoma (18 females, 28 males) (Table 1-1). 9 additional samples taken for another manuscript (6 healthy controls, 3 advanced adenoma samples, Table 1-1) were also used in the test sets for the MLG-based carcinoma classifier (Fig. 1d). So far, no study has investigated the given topic in a comparable manner; therefore no formal power analysis for sample size calculation could be performed. However, judging from previous 16S- and metagenomic shotgun-sequencing studies on the faecal microbiota in diseases, this is a reasonable sample size. Subjects were stratified with respect to gender, age and body mass index (BMI) so that the three groups (control, advanced adenoma, carcinoma) were comparable with respect to these variables. In the advanced adenoma group, 14 were located to the right colon (including caecum, ascending colon and transverse colon), 15 were located to the left colon (ranging from the splenic flexure to the sigmoid) and 15 to the rectum. In the carcinoma group, 8 were located to the right colon, 11 to the left colon and 27 to the rectum. Colorectal carcinoma was classified by the American Joint Committee on Cancer (AJCC) TNM staging system (Greene, F. L. Current TNM staging of colorectal cancer. Lancet. Oncol.8, 572-3 (2007)).

**Table 1-1: Clinical data of all 156 samples**

| Sample ID | Clinical data | | | | | |
|---|---|---|---|---|---|---|
| | State | Age (yrs) | BMI | TNM - Classification of carcinoma | Histology | Localization in colon (right/ left/rectum) |
| 147 samples | | | | | | |
| 31766 | controls | 73 | 32 | n.a. | n.a. | n.a. |
| 31537 | controls | 70 | 31 | n.a. | n.a. | n.a. |
| 31600 | controls | 70 | 30 | n.a. | n.a. | n.a. |
| 31428 | controls | 69 | 30 | n.a. | n.a. | n.a. |
| 530167 | controls | 72 | 30 | n.a. | n.a. | n.a. |
| 530315 | controls | 75 | 31 | n.a. | n.a. | n.a. |
| 530050 | controls | 71 | 29 | n.a. | n.a. | n.a. |
| 31160 | controls | 67 | 30 | n.a. | n.a. | n.a. |
| 530177 | controls | 71 | 29 | n.a. | n.a. | n.a. |
| 31700 | controls | 72 | 28.7 | n.a. | n.a. | n.a. |
| 31714 | controls | 69 | 28.3 | n.a. | n.a. | n.a. |
| 31219 | controls | 74 | 28.3 | n.a. | n.a. | n.a. |
| 31557 | controls | 74 | 28.5 | n.a. | n.a. | n.a. |
| 530154 | controls | 74 | 28.5 | n.a. | n.a. | n.a. |
| 530444 | controls | 62 | 22.1 | n.a. | n.a. | n.a. |
| 31021 | controls | 60 | 22.1 | n.a. | n.a. | n.a. |
| 530074 | controls | 68 | 22.1 | n.a. | n.a. | n.a. |
| 530227 | controls | 68 | 22.4 | n.a. | n.a. | n.a. |
| 530394 | controls | 67 | 22.58 | n.a. | n.a. | n.a. |
| 530295 | controls | 70 | 22.67 | n.a. | n.a. | n.a. |
| 530368 | controls | 70 | 22.8 | n.a. | n.a. | n.a. |
| 31300 | controls | 68 | 21.7 | n.a. | n.a. | n.a. |
| 31452 | controls | 66 | 21.7 | n.a. | n.a. | n.a. |
| 31723 | controls | 69 | 22.4 | n.a. | n.a. | n.a. |
| 531424 | controls | 46 | 28.74 | n.a. | n.a. | n.a. |
| 31009 | controls | 68 | 32 | n.a. | n.a. | n.a. |
| 530251 | controls | 72 | 31.8 | n.a. | n.a. | n.a. |
| 31416 | controls | 72 | 31.8 | n.a. | n.a. | n.a. |
| 530119 | controls | 70 | 31.6 | n.a. | n.a. | n.a. |
| 31749 | controls | 70 | 31.25 | n.a. | n.a. | n.a. |
| 530364 | controls | 63 | 31.17 | n.a. | n.a. | n.a. |
| 31328 | controls | 63 | 30.9 | n.a. | n.a. | n.a. |
| 530163 | controls | 63 | 30.4 | n.a. | n.a. | n.a. |
| 31379 | controls | 63 | 30.4 | n.a. | n.a. | n.a. |
| 31112 | controls | 66 | 30.3 | n.a. | n.a. | n.a. |
| 31750 | controls | 66 | 34.04 | n.a. | n.a. | n.a. |
| 530451 | controls | 68 | 29.8 | n.a. | n.a. | n.a. |
| 31129 | controls | 73 | 29.7 | n.a. | n.a. | n.a. |
| 530052 | controls | 64 | 29.38 | n.a. | n.a. | n.a. |
| 31512 | controls | 64 | 29.3 | n.a. | n.a. | n.a. |
| 31236 | controls | 65 | 29.06 | n.a. | n.a. | n.a. |
| 31360 | controls | 71 | 29.04 | n.a. | n.a. | n.a. |
| 31343 | controls | 72 | 29.03 | n.a. | n.a. | n.a. |
| 530215 | controls | 65 | 31.22 | n.a. | n.a. | n.a. |
| 31519 | controls | 65 | 28.90 | n.a. | n.a. | n.a. |
| 31450 | controls | 67 | 23.14 | n.a. | n.a. | n.a. |
| 531414 | controls | 46 | 23.15 | n.a. | n.a. | n.a. |
| 31071 | controls | 68 | 23.45 | n.a. | n.a. | n.a. |
| 530331 | controls | 70 | 23.67 | n.a. | n.a. | n.a. |
| 530258 | controls | 65 | 22.03 | n.a. | n.a. | n.a. |
| 31333 | controls | 68 | 22.2 | n.a. | n.a. | n.a. |
| 31232 | controls | 70 | 22.64 | n.a. | n.a. | n.a. |
| 530055 | controls | 64 | 22.78 | n.a. | n.a. | n.a. |
| 31267 | controls | 67 | 22.79 | n.a. | n.a. | n.a. |
| 31711 | controls | 66 | 30.02 | n.a. | n.a. | n.a. |
| 530075 | controls | 63 | 34.14 | n.a. | n.a. | n.a. |
| 31637 | controls | 66 | 29.98 | n.a. | n.a. | n.a. |
| 31398 | advanced adenoma | 68 | 34.01 | n.a. | n.a. | right |
| 531403 | advanced adenoma | 59 | 29.2 | n.a. | n.a. | left |
| 530168 | advanced adenoma | 62 | 32.8 | n.a. | n.a. | right |
| 530185 | advanced adenoma | 52 | 32.8 | n.a. | n.a. | right |
| 530600 | advanced adenoma | 70 | 25.2 | n.a. | n.a. | right |
| 31477 | advanced adenoma | 78 | 31.24 | n.a. | n.a. | rectum |
| 530403 | advanced adenoma | 75 | 30.12 | n.a. | n.a. | right |
| 530002 | advanced adenoma | 60 | 30 | n.a. | n.a. | right |
| 530697 | advanced adenoma | 63 | 30.47 | n.a. | n.a. | left |
| 31455 | advanced adenoma | 72 | 27.43 | n.a. | n.a. | rectum |
| 530756 | advanced adenoma | 70 | 29.38 | n.a. | n.a. | left |
| 31424 | advanced adenoma | 68 | 24.84 | n.a. | n.a. | right |
| 31501 | advanced adenoma | 69 | 24.4 | n.a. | n.a. | right |
| 31256 | advanced adenoma | 69 | 24.13 | n.a. | n.a. | left |
| 530297 | advanced adenoma | 78 | 24 | n.a. | n.a. | rectum |
| 530142 | advanced adenoma | 61 | 22.8 | n.a. | n.a. | left |
| 530026 | advanced adenoma | 83 | 21.94 | n.a. | n.a. | left |
| 530558 | advanced adenoma | 56 | 22.8 | n.a. | n.a. | rectum |
| 530054 | advanced adenoma | 64 | 20.52 | n.a. | n.a. | left |
| 530743 | advanced adenoma | 66 | 22.65 | n.a. | n.a. | rectum |
| 530867 | advanced adenoma | 58 | 27.02 | n.a. | n.a. | right |
| 530705 | advanced adenoma | 77 | 37.56 | n.a. | n.a. | rectum |
| 31337 | advanced adenoma | 62 | 35.43 | n.a. | n.a. | left |
| 31030 | advanced adenoma | 70 | 34.11 | n.a. | n.a. | rectum |
| 31282 | advanced adenoma | 63 | 34.08 | n.a. | n.a. | rectum |
| 530028 | advanced adenoma | 67 | 33.23 | n.a. | n.a. | right |
| 31449 | advanced adenoma | 63 | 30.77 | n.a. | n.a. | left |
| 31275 | advanced adenoma | 62 | 30.1 | n.a. | n.a. | right |
| 530018 | advanced adenoma | 56 | 29.41 | n.a. | n.a. | right |
| 530262 | advanced adenoma | 64 | 29.3 | n.a. | n.a. | left |
| 530039 | advanced adenoma | 67 | 28.34 | n.a. | n.a. | left |
| 530172 | advanced adenoma | 63 | 27.4 | n.a. | n.a. | rectum |
| 31137 | advanced adenoma | 67 | 27.14 | n.a. | n.a. | right |
| 31431 | advanced adenoma | 71 | 27.04 | n.a. | n.a. | left |
| 31233 | advanced adenoma | 62 | 26.88 | n.a. | n.a. | left |
| 530398 | advanced adenoma | 80 | 26.47 | n.a. | n.a. | left |
| 31582 | advanced adenoma | 77 | 25.7 | n.a. | n.a. | rectum |
| 530450 | advanced adenoma | 48 | 25.61 | n.a. | n.a. | rectum |
| 530623 | advanced adenoma | 62 | 24.3 | n.a. | n.a. | rectum |
| 530323 | advanced adenoma | 71 | 24.8 | n.a. | n.a. | rectum |
| 530348 | advanced adenoma | 58 | 21.7 | n.a. | n.a. | rectum |
| 530041 | advanced adenoma | 69 | 21.46 | n.a. | n.a. | left |
| 31705 | advanced adenoma | 84 | 24.7 | n.a. | n.a. | rectum |
| 530840 | advanced adenoma | 74 | 24.13 | n.a. | n.a. | right |
| 31446 | carcinoma | 84 | 31.22 | pTis | carcinoma in situ | right |
| 31881 | carcinoma | 60 | 30.08 | T4N1M0 | adenocarcinoma | rectum |
| 31866 | carcinoma | 73 | 29.75 | T3N0M0 | adenocarcinoma | left |
| 31874 | carcinoma | 74 | 29.17 | T4N1M1 | adenocarcinoma | left |
| 31549 | carcinoma | 84 | 27.77 | T1N0M0 | adenocarcinoma | left |
| 31878 | carcinoma | 52 | 27.14 | T3N1M0 | adenocarcinoma | rectum |
| 531281 | carcinoma | 72 | 27.12 | pTis | carcinoma in situ | left |
| 31877 | carcinoma | 72 | 26.67 | T3N1M0 | adenocarcinoma | rectum |
| 530373 | carcinoma | 47 | 25.78 | T2N0M0 | adenocarcinoma | rectum |
| 531155 | carcinoma | 72 | 24.22 | T1N0M0 | adenocarcinoma | right |
| 31868 | carcinoma | 64 | 24.09 | T2N0M0 | adenocarcinoma | rectum |
| 531361 | carcinoma | 72 | 23.83 | T4N1M0 | adenocarcinoma | rectum |
| 531775 | carcinoma | 77 | 23.50 | T3N0M0 | adenocarcinoma | right |
| 31865 | carcinoma | 55 | 23.34 | T2N0M0 | adenocarcinoma | rectum |
| 31223 | carcinoma | 65 | 23.31 | T4N0M0 | adenocarcinoma | rectum |
| 531469 | carcinoma | 45 | 22.15 | T2N0M0 | adenocarcinoma | rectum |
| 31876 | carcinoma | 56 | 20.07 | T3N2M0 | adenocarcinoma | rectum |
| 531416 | carcinoma | 63 | 33.56 | T2N0M0 | adenocarcinoma | rectum |
| 31872 | carcinoma | 46 | 30.86 | pTis | carcinoma in situ | left |
| 31276 | carcinoma | 82 | 30.47 | pTis | carcinoma in situ | right |
| 531333 | carcinoma | 69 | 29.94 | T3N1M0 | adenocarcinoma | left |
| 531382 | carcinoma | 65 | 29.74 | T3N0M0 | adenocarcinoma | rectum |
| 530890 | carcinoma | 77 | 29.41 | pTis | carcinoma in situ | rectum |
| 31237 | carcinoma | 67 | 29.40 | T3N0M0 | adenocarcinoma | right |
| 31004 | carcinoma | 64 | 29.35 | T1N0M0 | adenocarcinoma | rectum |
| 531277 | carcinoma | 64 | 29.07 | T1N0M0 | adenocarcinoma | rectum |
| 31871 | carcinoma | 72 | 28.41 | T2N0M0 | adenocarcinoma | left |
| 31188 | carcinoma | 65 | 28.03 | n.a. | adenocarcinoma | rectum |
| 31875 | carcinoma | 58 | 27.76 | T2N0M0 | adenocarcinoma | rectum |
| 31884 | carcinoma | 54 | 27.34 | pTis | carcinoma in situ | rectum |
| 31285 | carcinoma | 84 | 27.28 | T3N0M0 | adenocarcinoma | rectum |
| 31489 | carcinoma | 60 | 26.64 | T2N0M0 | adenocarcinoma | rectum |
| 31685 | carcinoma | 74 | 26.37 | T1N0M0 | adenocarcinoma | left |
| 531248 | carcinoma | 79 | 26.30 | T3N1M0 | adenocarcinoma | left |
| 31870 | carcinoma | 63 | 25.88 | T1N0M0 | adenocarcinoma | left |
| 531128 | carcinoma | 52 | 25.73 | T3N1M0 | adenocarcinoma | rectum |
| 31873 | carcinoma | 73 | 24.82 | T3N0M0 | adenocarcinoma | rectum |
| 31880 | carcinoma | 74 | 24.34 | T4N2M0 | adenocarcinoma | right |
| 531766 | carcinoma | 72 | 24.34 | T2N0M0 | adenocarcinoma | right |
| 31883 | carcinoma | 43 | 22.68 | T3N0M0 | adenocarcinoma | rectum |
| 531352 | carcinoma | 66 | 22.60 | T1N0M0 | carcinoma in situ | left |
| 31367 | carcinoma | 86 | 20.50 | T2N0M0 | adenocarcinoma | right |
| 31493 | carcinoma | 68 | 32.25 | pTis | carcinoma in situ | rectum |
| 31879 | carcinoma | 71 | 29.74 | T4N2M0 | adenocarcinoma | rectum |
| 531274 | carcinoma | 77 | 19.16 | T3N2M0 | adenocarcinoma | rectum |
| 31159 | carcinoma | 73 | 17.99 | T3N0M0 | adenocarcinoma | rectum |

| 9 additional samples taken for another manuscript | | | | | | |
|---|---|---|---|---|---|---|
| 532749 | controls | 55 | 27.76 | n.a. | n.a. | n.a. |
| 532779 | controls | 70 | 28.72 | n.a. | n.a. | n.a. |
| 532796 | controls | 73 | 26.56 | n.a. | n.a. | n.a. |
| 532802 | controls | 68 | 23.53 | n.a. | n.a. | n.a. |
| 532826 | controls | 78 | 31.22 | n.a. | n.a. | n.a. |
| 532832 | advanced adenoma | 68 | 27.55 | n.a. | n.a. | n.a. |
| 532305 | advanced adenoma | 56 | 43.58 | n.a. | n.a. | n.a. |
| 532915 | controls | 43 | 22.65 | n.a. | n.a. | n.a. |
| 531663 | advanced adenoma | 63 | 25.65 | n.a. | n.a. | n.a. |

### 1.2 Stool samples

Fresh stool samples were collected from all patients and subjects. Samples were mechanically homogenized with a sterile spatula, then 4 aliquots were taken, using the Sarstedt stool sampling system (Sarstedt, Nümbrecht, Germany). Each aliquot contained 1g of stool in a sterile 12ml cryovial. Fecal aliquots were then stored at home freezers at -20°C and transported to the laboratory within 48 hours after collection in a freezer pack, where they were immediately stored at -80°C. Patients and subjects did not receive probiotics or antibiotics within the last 3 months.

### 1.3 DNA extraction

Fecal samples were thawed on ice and DNA extraction was performed using the QiagenQIAamp DNA Stool Mini Kit (Qiagen) according to manufacturer's instructions. Extracts were treated with DNase-free RNase to eliminate RNA contamination. DNA quantity was determined using NanoDrop spectrophotometer, QubitFluorometer (with the Quant-iTTMdsDNA BR Assay Kit) and gel electrophoresis.

### 1.4 Metagenomic sequencing and gene catalogue construction

Paired-end metagenomic sequencing was performed on the Illumina platform (insert size 350bp, read length 100bp), and the sequencing reads were quality-controlled and *de novo* assembled into contigs using SOAPdenovo v2.04 (default parameters except for -K 51 -M 3 -F -u) (Luo, R. et al. SOAPdenovo2: an empirically improved memory-efficient short-read de novo assembler. Gigascience1, 18 (2012)), as described previously (Qin et al. 2012, *supra).* The high-quality sequencing reads (5 GB per sample on average) were assembled *de novo,* and the genes identified were compiled into a non-redundant catalogue of 3.5M genes, which allowed on average 76.3% of the reads in each sample to be mapped.

Gene prediction from the assembled contigs was performed using GeneMark v2.7d. Redundant genes were removed using BLAT (Kent, W. J. BLAT--the BLAST-like alignment tool. Genome Res.12, 656-64 (2002)) with the cutoff of 90% overlap and 95% identity (no gaps allowed). Relative abundances of the genes were determined by aligning high-quality sequencing reads to the gene catalogue using the same procedure as in Qin et al. 2012, *supra.*

Taxonomic assignment of the predicted genes was performed according to the IMG database (v400) using an in-house pipeline detailed previously (Qin et al. 2012, *supra*)*,* with 80% overlap and 65% identity, top 10% scores (BLASTN v2.2.24, -e 0.01 -b 100 -K 1 -F T -m 8). The cutoffs were 65% identity for assignment to phylum, 85% identity to genus, 95% identity to species; and ≥ 50% consensus for the taxon under question, if multiple hits remained.

### 2. Metagenome-wide association study (MGWAS)

For comparison of the faecal microbiome in healthy controls, advanced adenoma and carcinoma patients, genes that showed significant difference in relative abundance between any of the two groups were identified (Benjamin-Hochberg q-value < 0.1, Kruskal-Wallis test). These marker genes were then clustered into MLGs according to their abundance variation across all three groups of samples, which allowed identification of microbial species characteristic of each group (Qin et al. 2012, *supra*). 9 of the 147 samples contained more than 20% *Escherichia* (2 controls, 2 adenoma, 5 carcinoma samples), and were only used subsequently in the test sets for the MLG-based carcinoma classifiers (Fig. 1d). 9 additional samples taken for another manuscript (6 healthy controls, 3 advanced adenoma samples, Table 1-1) were also used in the test sets for the classifiers.

Taxonomic assignment and abundance profiling of the MLGs were performed according to the taxonomy and the relative abundance of their constituent genes, as previously described (Qin et al. 2012, *supra*)*.* Briefly, assignment to species requires more than 90% of genes in an MLG to align with the species' genome with more than 95% identity, 70% overlap of query. Assigning an MLG to a genus requires more than 80% of its genes to align with a genome with 85% identity in both DNA and protein sequences.

To explore signatures of the gut microbiome in healthy or tumour samples, the inventors identified 130,715 genes that displayed significant abundance differences in any two of the three groups (Kruskal-Wallis test,Benjamin-Hochberg q-value < 0.1). None of the available phenotypes other than tumour status displayed a significant difference among the controls, adenoma, and carcinoma patients, except for serum ferritin and red meat consumption (p < 0.05, Kruskal-Wallis test, Table 1-2). 58.9% of the gene markers were significantly elevated in carcinoma compared to both healthy and advanced adenoma samples, indicating that they were specific to colorectal cancer; another 24.3% of the genes were significantly more abundant in carcinomas than controls, with intermediate levels in advanced adenomas. Among the genes with a descending trend, 5388 (4.1% of total) were significantly reduced in carcinoma compared to both healthy and advanced adenoma samples; 2601 (2.0% of total) were significantly less abundant in carcinomas than controls, with intermediate levels in advanced adenomas. These control-enriched genes were more often mapped to Kyoto Encyclopedia of Genes and Genomes (KEGG) pathways than the adenoma- or carcinoma-enriched genes. The disparity in the number of increasing and decreasing genes suggests that the increase in pathobionts is more pronounced than the decrease in beneficial bacteria during the development of carcinoma. The significantly different genes were clustered into 126 MLGs according to their abundance co-variations among all samples, which allowed identification of microbial species characteristic of each group (Qin et al. 2012, *supra*).

### 3. MLG-based classification of colorectal carcinoma or adenoma

To illustrate diagnostic value of the faecal microbiome for colorectal cancer, the inventors constructed a random forest classifier that could detect carcinoma samples. A random forest model (R.2.14, randomForest4.6-7 package) (Liaw, Andy & Wiener, Matthew. Classification and Regression by randomForest, R News (2002), Vol. 2/3 p. 18) was trained using the MLG abundance profile of the training cohort (set) to select the optimal set of MLG markers. The model was tested on the testing set and the prediction error was determined. About the random forest model, using "randomForest4.6-7 package" package in R vision 2.14, input was a training dataset (namely relative abundance profiles of selected MLGs in training samples), sample disease status (sample disease status of training samples was a vector, 1 for case, 0 for control), and a testing dataset (just the relative abundance profiles of selected MLGs in testing set). Then the inventors used the random Forest function from randomForest package in R software to build the classification, and predict function was used to predict the testing set. Output was the prediction results (probability of illness; cut-off was the best cut-off and if the probability of illness ≥the best cut-off, the subject was at risk of illness).

10-fold cross-validation was performed on a random forest model (R 3.0.2, randomForest4.6-7 package) using the MLG abundance profile of the control, advanced adenoma or carcinoma samples. The cross-validational error curves (average of 10 test sets each) from 5 trials of the 10-fold cross-validation were averaged, and the minimum error in the averaged curve plus the standard deviation at that point was used as the cutoff. All sets (≤ 50) of MLG markers with an error less than the cutoff were listed, and the set with the smallest number of MLGs was chosen as the optimal set. The probability of adenoma or carcinoma was calculated using this set of MLGs and an ROC was drawn (R 3.0.2, pROC3 package). The model was further tested on the testing set and the prediction error was determined.

5 repeats of 10-fold cross-validation (i.e. 50 tests) in the training set consisting of 55 controls and 41 carcinoma samples (Table 1-1) led to the optimal selection of 15 MLG markers (Table 2-1, Table 2-2, Table 3). Briefly, 5 repeats of 10-fold cross-validation (i.e. 50 tests) were carried out in the training set consisting of 55 controls and 41 carcinoma samples. Random forest test sorted the importance of MLGs in each time. The inventors picked the top 15 important MLG together, and sorted the mlg by their occurance times. The top 15 MLGs were used to construct the classifier. The sort of MLG importance was listed in Table 6.

Our results showed that the 15 MLGs performed nicely on the training set, with an area under receiver operating curve (AUC) of 98.34% (cut-off=0.5, Fig. 1a, 1b, 1c, Table 4-1, Table 4-2, Table 5 and Table 6). The classification error remained low on the test set (8 controls, 47 advanced adenomas and 5 carcinomas), showing an area under receiver operating curve (AUC) of 96% (advanced adenoma considered as non-carcinoma, cut-off=0.5, Fig. 1d, 1e, Table 5), in agreement with their mostly benign nature. Among the MLG markers were the likely oral anaerobes mlg-75 and mlg-84, the former also showed a high odds ratio for adenoma (Table 2-2), suggesting an early role in pathogenesis. Other MLG markers included *Bacteroides massiliensis,* mlg-2985, mlg-121 and 10 further taxonomically undefined MLGs (Table 2-2). Thus, MLGs selected by the carcinoma classifier captured important features of the deteriorating gut microbiome in adenoma and carcinoma and have great potential for early and non-invasive diagnosis of these tumours.

In addition, the results in Table 6 showed that the AUC is 0.91751663 for the combination of the top 2 important MLGs (MLG 5045 and MLG 121); 0.970731707 for the combination of the top 3 important MLGs (MLG 5045, MLG 121 and MLG 75); 0.959645233 for the top 4 MLGs; 0.975609756 for the top 5 MLGs; 0.978713969 for the top 6 MLGs; 0.980044346 for the top 7 MLGs; 0.985365854 for the top 8 MLGs; 0.984035477 for the top 9 MLGs; 0.981818182 for the top 10 MLGs; 0.980931264 for the top 11 MLGs; 0.979157428 for the top 12 MLGs; 0.987583149 for the top 13 MLGs; 0.986696231 for the top 14 MLGs; and 0.983370288 for the 15 MLGs. These results suggested that MLG 5045 and MLG 121 are the most important biomarkers among the 15 MLGs, and the combination of MLG 5045 and MLG 121 is sufficient for diagnosis of colorectal carcinoma and/or determining the risk of colorectal carcinoma with high sensitivity and high specificity (AUC=0.91751663); and that incorporation of other MLG biomarkers of the 15 MLGs may improve the sensitivity and specificity of the diagnosis or prediction to a certain extent. In particular, these results also suggested that the combination of the top 13 MLGs may be considered as the optimal biomarker set, which has the best sensitivity and specificity (AUC=0.987583149) for the diagnosis or prediction.

These results fully supported that the MLGs as identified herein (in particular, MLG 5045 and MLG 121, optionally combined with one or more of the other MLGs of the 15 MLGs) could be used as biomarkers for diagnosis of colorectal carcinoma and/or determining the risk of colorectal carcinoma with high sensitivity and high specificity.

The inventors further directly investigated the utility of the gut MLGs for identifying adenoma, which is more difficult to screen than colorectal carcinoma but important for early intervention.

Similarly, 5 repeats of 10-fold cross-validation (i.e. 50 tests) were carried out in the training set consisting of 55 controls and 42 adenoma samples. Random forest test sorted the importance of MLGs in each time. The inventors picked the top 10 important MLG together, and sorted the mlg by their occurance times. The top 10 MLG were used to construct the classifier. The sort of MLG importance was listed in Table 11.

After 5 repeats of 10-fold cross-validation, the random forest model chose 10 MLGs (Table 7-1, Table 7-2, Fig.2a) that allowed optimal classification of the training set (55 controls and 42 advanced adenoma, Table 9 and Table 10, Fig. 2b), with an AUC of 0.8738 (cut-off=0.5, Fig.2c). On the test set (unused new samples consisting of 15 controls and 15 advanced adenoma), all the advanced adenoma samples were correctly classified (cut-off=0.4572, Fig. 2d, 2e, Table 10). Therefore, the faecal MLGs offer new opportunities for non-invasive detection of colorectal advanced adenoma.

In addition, the results in Table 11 showed that the AUC is 0.782251082 for the combination of the top 2 important MLGs (MLG 317 and MLG 3770); 0.805194805 for the combination of the top 3 important MLGs (MLG 317, MLG 3770 and MLG 3840); 0.773160173 for the top 4 MLGs; 0.795238095 for the top 5 MLGs; 0.780952381 for the top 6 MLGs; 0.895670996 for the top 7 MLGs; 0.896536797 for the top 8 MLGs; 0.884848485 for the top 9 MLGs; and 0.873809524 for the 10 MLGs. These results suggested that MLG 317, MLG 3770 and MLG 3840 are the most important biomarkers among the 10 MLGs, and the combination of MLG 317, MLG 3770 and MLG 3840 is sufficient for diagnosis of colorectal advanced adenoma and/or determining the risk of colorectal advanced adenoma with high sensitivity and high specificity (AUC=0.805194805); and that incorporation of other MLG biomarkers of the 10 MLGs may improve the sensitivity and specificity of the diagnosis or prediction to a certain extent. In particular, these results also suggested that the combination of the top 8 MLGs may be considered as the optimal biomarker set, which has the best sensitivity and specificity (AUC=0.896536797) for the diagnosis or prediction.

These results fully supported that the MLGs as identified herein (in particular, MLG 317, MLG 3770 and MLG 3840, optionally combined with one or more of the other MLGs of the 10 MLGs) could be used as biomarkers for diagnosis of colorectal advanced adenoma and/or determining the risk of colorectal advanced adenoma with high sensitivity and high specificity.

**Table 1-2: Concordance in phenotypes among the three groups in Table 1-1.**

| **Phenotype** | **Average in controls** | **Average in advanced adenoma** | **Average in carcinoma** | **p-value (Kruskal-Wallis)** | **p.adjust (BH)** | **# samples used (138 max)** |
|---|---|---|---|---|---|---|
| **Age (yrs)** | 67.44 | 66.19 | 66.10 | 0.3936 | 0.6332 | 138 |
| **BMI** | 27.50 | 27.82 | 26.82 | 0.4735 | 0.6332 | 138 |
| **Waist (cm)** | 100.78 | 99.53 | 99.56 | NA | NA | 108 |
| **Hip (cm)** | 105.35 | 105.97 | 102.29 | NA | NA | 100 |
| **WHR (waist-hip-ratio)** | 0.94 | 0.93 | 0.97 | 0.0968 | 0.4768 | 132 |
| **GGT (U/L)** | 45.07 | 49.98 | 38.22 | 0.8623 | 0.8623 | 138 |
| **GOT (AST) (U/L)** | 23.45 | 23.69 | 24.24 | 0.6200 | 0.7188 | 138 |
| **GPT (ALT) (U/L)** | 26.13 | 27.86 | 23.59 | 0.2877 | 0.6332 | 138 |
| **Fasting insulin (µU/mL)** | 10.44 | 10.48 | 9.53 | NA | NA | 68 |
| **Fasting glucose (mg/L)** | 109.73 | 107.45 | 103.48 | 0.3640 | 0.6332 | 137 |
| **HOMA index** | 2.97 | 2.83 | 2.55 | NA | NA | 68 |
| **HbalC (%)** | 5.83 | 5.96 | 5.93 | NA | NA | 95 |
| **CRP (mg/L)** | 0.66 | 0.61 | 1.02 | 0.3177 | 0.6332 | 138 |
| **Serum ferritin (ng/mL)** | 234.41 | 238.48 | 126.94 | 0.0004 | 0.0080 | 136 |
| **Hb (g/L)** | 14.67 | 16.88 | 14.07 | 0.2585 | 0.6332 | 138 |
| **TG (mg/L)** | 123.49 | 133.52 | 128.63 | 0.6829 | 0.7188 | 136 |
| **HDL (mg/L)** | 59.64 | 65.24 | 57.88 | 0.1148 | 0.4768 | 136 |
| **LDL (mg/L)** | 146.79 | 143.19 | 138.46 | 0.6825 | 0.7188 | 136 |
| **Red meat (g/wk)** | 110.85 | 150.64 | 141.00 | 0.0483 | 0.4768 | 132 |
| **Wite meat (g/wk)** | 77.26 | 81.79 | 64.92 | 0.4619 | 0.6332 | 132 |
| **Total meat (g/wk)** | 188.87 | 234.67 | 207.53 | 0.1192 | 0.4768 | 132 |
| **Fish (g/wk)** | 151.85 | 171.43 | 166.67 | 0.6123 | 0.7188 | 132 |
| **Vegetables (g/wk)** | 1740.74 | 1690.48 | 1461.11 | 0.4749 | 0.6332 | 132 |
| **Fruits (g/wk)** | 1798.15 | 1604.76 | 1397.22 | 0.3733 | 0.6332 | 132 |
| **Fiber intake (g/wk)** | 50.17 | 64.08 | 66.69 | 0.2600 | 0.6332 | 132 |

**Table 2-1: 15 most discriminant MLGs (species markers) associated with colorectal carcinoma (enrichment: according to MLG direction of enrichment in control (CTRL) vs. adenoma (AA), control (CTRL) vs. carcinoma (CRC), and adenoma (AA) vs. carcinoma (CRC), where + indicates enrichment in latter, - indicates enrichment in former, and 0 indicates no difference (p ≥ 0.05, Wilcoxon-rank sum test, Bonferroni correction to control for multiple testing).**

| MLG ID | p-value (Kruskal) | p.adjust (BH) | p-value (Wilcoxon) | | | Enrich ment | Classifier | | Rank mean | | | Occurrence | | |
|---|---|---|---|---|---|---|---|---|---|---|---|---|---|---|
| | | | CTRL vs AA | CTRL vs CRC | AA vs CRC | | CRC | AA | CTRL | AA | CRC | CTRL | AA | CRC |
| 317 | 3.15E-07 | 9.64E-06 | 1.65E-03 | 9.00E-07 | 4.12E-02 | +,+,+ | YES | YES | 49 | 74 | 93 | 0.51 | 0.88 | 1.00 |
| 2985 | 1.16E-05 | 9.17E-05 | 4.35E-01 | 2.44E-06 | 2.77E-02 | 0,+,+ | YES | NO | 54 | 67 | 92 | 0.47 | 0.57 | 0.93 |
| 75 | 3.81E-09 | 6.05E-07 | 1.13E-01 | 1.65E-08 | 1.83E-04 | 0,+,+ | YES | NO | 54 | 64 | 95 | 0.11 | 0.26 | 0.63 |
| 84 | 3.19E-08 | 2.55E-06 | 3.63E-01 | 1.38E-07 | 8.98E-05 | 0,+,+ | YES | NO | 54 | 63 | 97 | 0.24 | 0.38 | 0.73 |
| 121 | 1.90E-09 | 3.68E-07 | 7.38E-01 | 2.88E-09 | 5.85E-05 | 0,+,+ | YES | NO | 53 | 62 | 100 | 0.27 | 0.36 | 0.90 |
| 131 | 1.84E-08 | 1.74E-06 | 3.60E-01 | 4.15E-08 | 4.81E-04 | 0,+,+ | YES | NO | 55 | 64 | 95 | 0.13 | 0.24 | 0.66 |
| 100 | 7.75E-06 | 7.27E-05 | 2.54E-01 | 6.80E-06 | 1.29E-02 | 0,+,+ | YES | NO | 57 | 67 | 90 | 0.15 | 0.29 | 0.59 |
| 109 | 1.60E-10 | 6.66E-08 | 7.99E-01 | 7.93E-10 | 1.50E-05 | 0,+,+ | YES | NO | 53 | 61 | 100 | 0.18 | 0.26 | 0.80 |
| 219 | 1.90E-09 | 3.68E-07 | 7.36E-01 | 5.61E-09 | 7.33E-05 | 0,+,+ | YES | NO | 54 | 62 | 98 | 0.18 | 0.26 | 0.76 |
| 223 | 5.22E-11 | 3.18E-08 | 8.37E-02 | 7.66E-10 | 4.97E-05 | 0,+,+ | YES | NO | 54 | 63 | 96 | 0.04 | 0.17 | 0.63 |
| 1564 | 6.39E-09 | 8.59E-07 | 7.16E-02 | 4.05E-08 | 2.95E-04 | 0,+,+ | YES | NO | 55 | 65 | 94 | 0.05 | 0.21 | 0.59 |
| 5045 | 1.31E-10 | 5.75E-08 | 2.89E-02 | 3.47E-10 | 2.24E-04 | +,+,+ | YES | NO | 52 | 65 | 97 | 0.05 | 0.24 | 0.73 |
| 114 | 9.32E-09 | 1.06E-06 | 1.76E-01 | 4.83E-09 | 1.08E-03 | 0,+,+ | YES | NO | 52 | 65 | 97 | 0.20 | 0.36 | 0.83 |
| 166 | 3.29E-12 | 5.21E-09 | 8.63E-01 | 6.92E-10 | 4.11E-07 | 0,+,+ | YES | NO | 55 | 59 | 100 | 0.09 | 0.17 | 0.73 |
| 135 | 8.34E-13 | 1.65E-09 | 1.00E+00 | 3.95E-09 | 2.36E-07 | 0,+,+ | YES | NO | 57 | 58 | 98 | 0.04 | 0.05 | 0.63 |

**Table 2-2: 15 most discriminant MLGs (species markers) associated with colorectal carcinoma**

| MLG ID | Abundance mean | | | Odds ratio (95% CI) | | Gene NO. | MLG annotation | Best aligned strain (nucleotide) | Fraction of genes aligned (>65% identity) | Average identity of genes aligned (%) |
|---|---|---|---|---|---|---|---|---|---|---|
| | CTRL | AA | CRC | CTRL vs AA | CTRL vs CRC | | | | | |
| 317 | 4.13 | 3.77 | 8.77 | 1.02 (0.68-1.53) | 1.28 (0.8-2.05) | 120 | Bacteroides massiliensis | Bacteroides massiliensis | 0.9583 | 99.54 |
| | E-07 | E-07 | E-07 | | | | | | | |
| 2985 | 1.44 | 1.20 | 3.03 | 1.07 (0.71-1.59) | 1.5 (0.93-2.43) | 1567 | mlg-2985 | Dialister invisus | 0.8826 | 98.49 |
| | E-06 | E-06 | E-06 | | | | | | | |
| 75 | 2.80 | 1.00 | 1.55 | 111.87 (0.13-99028.45) | 4.78967603662182e+243 (5.04358781846618e+37-Inf) | 178 | mlg-75 | Fusobacterium sp. oral taxon 370 | 0.7753 | 99.36 |
| | E-09 | E-10 | E-07 | | | | | | | |
| 84 | 2.30 | 1.10 | 8.44 | 1.37 (0.84-2.25) | 254129973946495 (36942.45-1.7481797484976 e+24) | 165 | mlg-84 | Gemella morbillorum | 0.5212 | 97.88 |
| | E-09 | E-09 | E-08 | | | | | | | |
| 121 | 5.08 | 4.81 | 6.93 | 2.54 (0.71-9.08) | 4011933781285.55 (18.76-8.58034239482951e+ 23) | 2630 | mlg-121 | Prevotella copri | 0.7293 | 98.07 |
| | E-07 | E-08 | E-06 | | | | | | | |
| 131 | 3.59 | 1.10 | 1.66 | 6.76 (0.2-225.78) | 2.61572518085332e+253 (9.3301722699787e+44-Inf) | 1184 | mlg-131 | Unclassified | | |
| | E-08 | E-09 | E-06 | | | | | | | |
| 100 | 4.19 | 1.10 | 3.00 | 5.41 (0.13-232.75) | Inf (1.18197401454276e+50-Inf) | 296 | mlg-100 | Unclassified | | |
| | E-08 | E-09 | E-06 | | | | | | | |
| 109 | 6.14 | 3.10 | 2.87 | 5.12 (0.43-61.37) | 9.22843866981152e+91 (189.69-4.48968677759076e +181) | 687 | mlg-109 | Unclassified | | |
| | E-08 | E-09 | E-06 | | | | | | | |
| 219 | 4.04 | 1.80 | 2.53 | 4.31 (0.25-75.58) | 6464869070139582 (0.7-5.9327349907021e+31) | 328 | mlg-219 | Unclassified | | |
| | E-08 | E-09 | E-07 | | | | | | | |
| 223 | 2.90 | 2.00 | 5.31 | 3.23 (0.33-31.58) | 4.18468389007774e+201 | 1407 | mlg-223 | Unclassified | | |
| | E-09 | E-10 | E-07 | | (868257309.25-Inf) | | | | | |
| 1564 | 1.52 | 5.00 | 8.54 | 1.77 (0.11-29.19) | 2.2802941587178e+217 (82198762116180.2-Inf) | 140 | mlg-1564 | Unclassified | | |
| | E-08 | E-10 | E-07 | | | | | | | |
| 5045 | 1.30 | 2.08 | 2.37 | 1.7 (0.86-3.39) | 3.13 (0.96-10.21) | 187 | mlg-5045 | Unclassified | | |
| | E-07 | E-08 | E-07 | | | | | | | |
| 114 | 2.17 | 5.14 | 9.26 | 1.39 (0.73-2.64) | 4.77 (0.62-36.99) | 562 | mlg-114 | Unclassified | | |
| | E-07 | E-08 | E-07 | | | | | | | |
| 166 | 3.00 | 7.00 | 3.10 | 0.88 (0.53-1.45) | 4.50269368364809e+58 (189.1-1.07213823045308e+ 115) | 345 | mlg-166 | Unclassified | | |
| | E-10 | E-10 | E-07 | | | | | | | |
| 135 | 4.00 | 4.82 | 1.02 | 0.59 (0.08-4.51) | 3.31 (0.71-15.34) | 1502 | mlg-135 | Unclassified | | |
| | E-09 | E-08 | E-06 | | | | | | | |

**Table 3: Sequences of the 15 MLG species**

| MLG ID | SEQ ID NO: | Genes Number |
|---|---|---|
| mlg_id:84 | 1-165 | 165 |
| mlg_id:131 | 166-1349 | 1184 |
| mlg_id:75 | 1350-1527 | 178 |
| mlg_id:114 | 1528-2089 | 562 |
| mlg_id:1564 | 2090-2229 | 140 |
| mlg_id:135 | 2230-3731 | 1502 |
| mlg_id:5045 | 3732-3918 | 187 |
| mlg_id:121 | 3919-6548 | 2630 |
| mlg_id:109 | 6549-7235 | 687 |
| mlg_id:166 | 7236-7580 | 345 |
| mlg_id:317 | 7581-7700 | 120 |
| mlg_id:219 | 7701-8028 | 328 |
| mlg_id:2985 | 8029-9595 | 1567 |
| mlg_id:100 | 9596-9891 | 296 |
| mlg_id:223 | 9892-11298 | 1407 |

**Table 4-1: 15 MLGs relative abundance profiles in 96 samples (training set)**

| number (NO. 1-41: CRC; NO. 42-96: CTRL) | Sample ID | MLG | | | | | | |
|---|---|---|---|---|---|---|---|---|
| | | 121 | 2985 | 135 | 223 | 131 | 109 | 114 |
| 1 | 31446 | 3.64E-08 | 3.71E-08 | 1.70E-08 | 0 | 6.57E-09 | 1.64E-09 | 9.39E-10 |
| 2 | 31881 | 4.53E-07 | 2.82E-09 | 4.47E-06 | 2.92E-11 | 8.90E-08 | 2.70E-08 | 6.33E-09 |
| 3 | 31866 | 9.87E-07 | 2.32E-06 | 1.79E-07 | 4.84E-08 | 4.27E-07 | 2.66E-07 | 1.14E-07 |
| 4 | 31874 | 5.28E-09 | 2.72E-09 | 8.47E-11 | 4.33E-06 | 1.79E-09 | 7.03E-11 | 3.88E-10 |
| 5 | 31549 | 1.56E-08 | 1.41E-06 | 1.37E-08 | 8.54E-10 | 3.31E-09 | 4.59E-09 | 3.27E-09 |
| 6 | 31878 | 4.35E-07 | 3.17E-06 | 0 | 3.40E-06 | 1.14E-08 | 9.08E-08 | 3.67E-08 |
| 7 | 531281 | 8.32E-09 | 6.04E-09 | 1.18E-09 | 3.73E-10 | 3.71E-09 | 3.97E-11 | 2.86E-10 |
| 8 | 31877 | 9.23E-09 | 8.07E-09 | 5.15E-08 | 1.38E-08 | 3.67E-09 | 0 | 2.72E-10 |
| 9 | 530373 | 4.42E-09 | 1.67E-06 | 0 | 8.96E-11 | 1.11E-09 | 0 | 0 |
| 10 | 531155 | 1.19E-07 | 4.10E-06 | 1.91E-08 | 1.02E-09 | 5.84E-08 | 2.61E-08 | 1.51E-08 |
| 11 | 31868 | 4.12E-05 | 2.20E-06 | 7.21E-06 | 4.96E-08 | 1.82E-05 | 1.23E-05 | 4.39E-06 |
| 12 | 531361 | 0.00011555 | 2.34E-08 | 1.70E-05 | 1.69E-09 | 4.75E-05 | 3.28E-05 | 1.21E-05 |
| 13 | 531775 | 4.10E-08 | 8.73E-06 | 6.04E-09 | 0 | 1.81E-08 | 7.30E-09 | 4.68E-09 |
| 14 | 31865 | 2.08E-08 | 1.25E-08 | 2.17E-09 | 3.67E-09 | 9.18E-09 | 1.41E-09 | 1.83E-09 |
| 15 | 31223 | 4.96E-07 | 6.64E-10 | 2.63E-07 | 0 | 7.32E-08 | 9.57E-08 | 2.75E-08 |
| 16 | 531469 | 4.07E-08 | 3.22E-08 | 4.27E-09 | 5.17E-07 | 1.71E-08 | 6.47E-09 | 4.59E-09 |
| 17 | 31876 | 3.29E-08 | 1.22E-08 | 3.55E-09 | 3.59E-07 | 1.53E-08 | 7.31E-09 | 4.81E-09 |
| 18 | 531416 | 1.77E-08 | 1.16E-05 | 3.74E-09 | 1.94E-07 | 6.71E-09 | 1.25E-09 | 1.40E-09 |
| 19 | 31872 | 8.12E-08 | 1.66E-05 | 1.82E-08 | 0 | 3.81E-08 | 1.93E-08 | 1.18E-08 |
| 20 | 31276 | 1.03E-06 | 3.80E-08 | 4.49E-06 | 0 | 2.72E-07 | 3.28E-07 | 5.37E-07 |
| 21 | 531333 | 2.06E-08 | 2.07E-08 | 4.14E-09 | 0 | 1.05E-08 | 2.75E-09 | 1.86E-09 |
| 22 | 531382 | 0 | 7.51E-09 | 0 | 0 | 0 | 0 | 0 |
| 23 | 530890 | 1.28E-09 | 0 | 0 | 1.46E-09 | 0 | 0 | 0 |
| 24 | 31237 | 1.64E-06 | 0 | 5.71E-08 | 0 | 5.17E-07 | 2.04E-07 | 2.66E-07 |
| 25 | 31004 | 0 | 0 | 0 | 0 | 0 | 0 | 0 |
| 26 | 531277 | 1.75E-08 | 1.03E-08 | 0 | 1.12E-08 | 0 | 1.40E-09 | 1.07E-09 |
| 27 | 31871 | 5.00E-09 | 1.46E-06 | 0 | 0 | 0 | 2.38E-09 | 2.14E-10 |
| 28 | 31188 | 1.37E-06 | 6.23E-09 | 1.36E-07 | 0 | 9.90E-09 | 3.48E-07 | 1.19E-07 |
| 29 | 31875 | 5.42E-10 | 3.45E-06 | 0 | 1.20E-08 | 0 | 0 | 0 |
| 30 | 31884 | 1.48E-08 | 2.63E-06 | 0 | 0 | 0 | 8.15E-09 | 4.46E-09 |
| 31 | 31285 | 3.27E-08 | 8.48E-09 | 4.04E-09 | 5.89E-09 | 0 | 1.35E-08 | 9.36E-09 |
| 32 | 31489 | 1.53E-06 | 2.71E-06 | 1.81E-11 | 5.33E-09 | 1.54E-09 | 1.16E-06 | 2.83E-07 |
| 33 | 31685 | 2.45E-08 | 1.73E-09 | 0 | 1.16E-09 | 0 | 1.83E-08 | 6.27E-09 |
| 34 | 531248 | 8.26E-05 | 1.06E-05 | 5.69E-10 | 0 | 1.18E-07 | 6.05E-05 | 1.47E-05 |
| 35 | 31870 | 9.25E-08 | 2.10E-08 | 3.45E-10 | 0 | 0 | 7.15E-08 | 2.13E-08 |
| 36 | 531128 | 2.88E-06 | 2.20E-08 | 7.93E-06 | 2.61E-08 | 8.09E-07 | 4.41E-07 | 2.64E-06 |
| 37 | 31873 | 0 | 1.45E-06 | 0 | 4.47E-06 | 0 | 0 | 0 |
| 38 | 31880 | 0 | 2.62E-05 | 0 | 0 | 0 | 0 | 0 |
| 39 | 531766 | 6.15E-08 | 2.31E-07 | 0 | 3.24E-08 | 0 | 1.46E-08 | 4.21E-09 |
| 40 | 31883 | 3.34E-05 | 1.14E-05 | 0 | 8.27E-06 | 2.44E-08 | 8.85E-06 | 2.65E-06 |
| 41 | 531352 | 1.25E-08 | 1.19E-05 | 0 | 2.17E-09 | 0 | 8.91E-10 | 9.75E-10 |
| 42 | 31766 | 0 | 6.09E-09 | 0 | 0 | 0 | 0 | 0 |
| 43 | 31537 | 3.10E-08 | 4.51E-06 | 0 | 0 | 0 | 1.60E-09 | 9.02E-10 |
| 44 | 31600 | 0 | 0 | 0 | 0 | 0 | 0 | 0 |
| 45 | 31428 | 3.09E-08 | 8.25E-10 | 0 | 0 | 6.76E-12 | 3.73E-09 | 9.41E-10 |
| 46 | 530167 | 2.88E-08 | 1.20E-09 | 0 | 2.61E-10 | 0 | 1.55E-09 | 8.91E-10 |
| 47 | 530315 | 3.71E-07 | 0 | 0 | 0 | 4.20E-09 | 8.85E-09 | 1.63E-09 |
| 48 | 530050 | 0 | 5.32E-06 | 0 | 9.12E-09 | 0 | 0 | 0 |
| 49 | 31160 | 1.42E-09 | 2.19E-09 | 0 | 0 | 0 | 0 | 1.78E-09 |
| 50 | 530177 | 0 | 2.02E-09 | 0 | 0 | 0 | 0 | 0 |
| 51 | 31700 | 0 | 4.27E-09 | 0 | 0 | 0 | 0 | 0 |
| 52 | 31714 | 6.40E-08 | 1.41E-08 | 0 | 0 | 2.07E-09 | 1.40E-09 | 9.32E-07 |
| 53 | 31219 | 0 | 2.64E-05 | 0 | 0 | 0 | 0 | 0 |
| 54 | 31557 | 0 | 3.95E-09 | 0 | 0 | 0 | 0 | 0 |
| 55 | 530154 | 3.16E-09 | 4.13E-08 | 0 | 0 | 0 | 0 | 0 |
| 56 | 530444 | 1.57E-06 | 0 | 0 | 0 | 1.50E-08 | 1.02E-07 | 7.99E-09 |
| 57 | 31021 | 0 | 0 | 0 | 0 | 0 | 0 | 0 |
| 58 | 530074 | 0 | 0 | 0 | 0 | 0 | 0 | 0 |
| 59 | 530227 | 0 | 0 | 0 | 0 | 0 | 0 | 0 |
| 60 | 530394 | 0 | 6.97E-07 | 0 | 0 | 0 | 0 | 0 |
| 61 | 530295 | 2.05E-08 | 0 | 0 | 0 | 2.88E-10 | 5.88E-10 | 1.46E-08 |
| 62 | 530368 | 0 | 1.27E-06 | 0 | 0 | 0 | 0 | 0 |
| 63 | 31300 | 0 | 0 | 0 | 0 | 0 | 0 | 0 |
| 64 | 31452 | 0 | 0 | 0 | 0 | 0 | 0 | 0 |
| 65 | 31723 | 0 | 0 | 0 | 0 | 0 | 0 | 0 |
| 66 | 531424 | 0 | 0 | 0 | 0 | 0 | 0 | 0 |
| 67 | 31009 | 0 | 0 | 0 | 0 | 0 | 0 | 0 |
| 68 | 530251 | 0 | 0 | 0 | 0 | 0 | 0 | 0 |
| 69 | 31416 | 0 | 1.10E-07 | 0 | 0 | 0 | 0 | 0 |
| 70 | 530119 | 0 | 0 | 0 | 0 | 0 | 0 | 0 |
| 71 | 31749 | 0 | 0 | 2.31E-06 | 0 | 0 | 0 | 0 |
| 72 | 530364 | 0 | 4.45E-06 | 0 | 0 | 0 | 0 | 0 |
| 73 | 31328 | 0 | 2.72E-07 | 0 | 0 | 0 | 0 | 0 |
| 74 | 530163 | 0 | 0 | 0 | 0 | 0 | 0 | 0 |
| 75 | 31379 | 1.55E-08 | 0 | 0 | 0 | 0 | 2.24E-10 | 2.15E-09 |
| 76 | 31112 | 1.93E-08 | 0 | 0 | 0 | 0 | 2.14E-10 | 2.61E-09 |
| 77 | 31711 | 0 | 2.96E-08 | 0 | 0 | 0 | 0 | 0 |
| 78 | 31750 | 0 | 0 | 0 | 0 | 0 | 0 | 0 |
| 79 | 530451 | 4.29E-07 | 1.26E-10 | 0 | 0 | 3.98E-08 | 4.88E-08 | 1.86E-06 |
| 80 | 31129 | 5.68E-08 | 2.63E-09 | 0 | 0 | 1.05E-09 | 0 | 0 |
| 81 | 530052 | 0 | 2.67E-09 | 0 | 0 | 0 | 0 | 0 |
| 82 | 31512 | 0 | 9.36E-06 | 0 | 0 | 0 | 0 | 0 |
| 83 | 31236 | 0 | 1.73E-09 | 0 | 0 | 0 | 0 | 0 |
| 84 | 31360 | 0 | 1.35E-05 | 0 | 0 | 0 | 0 | 0 |
| 85 | 31343 | 0 | 1.66E-09 | 0 | 0 | 0 | 0 | 0 |
| 86 | 530215 | 0 | 7.59E-10 | 0 | 0 | 0 | 0 | 0 |
| 87 | 31519 | 0 | 0 | 3.41E-07 | 0 | 0 | 0 | 0 |
| 88 | 31450 | 0 | 0 | 0 | 0 | 0 | 0 | 0 |
| 89 | 531414 | 0 | 0 | 0 | 0 | 0 | 0 | 0 |
| 90 | 31071 | 0 | 0 | 0 | 0 | 0 | 0 | 0 |
| 91 | 530331 | 4.72E-10 | 0 | 0 | 0 | 0 | 0 | 0 |
| 92 | 530258 | 0 | 0 | 0 | 0 | 0 | 0 | 0 |
| 93 | 31333 | 0 | 0 | 0 | 0 | 0 | 0 | 0 |
| 94 | 31232 | 0 | 0 | 0 | 0 | 0 | 0 | 0 |
| 95 | 530055 | 4.15E-10 | 0 | 0 | 0 | 0 | 0 | 0 |
| 96 | 31267 | 0 | 0 | 0 | 0 | 0 | 0 | 0 |

**Table 4-2: 15 MLGs relative abundance profiles in 96 samples (training set)**

| number (NO. 1-41: CRC; NO. 42-96: CTRL) | Sample ID | MLG | | | | | | | |
|---|---|---|---|---|---|---|---|---|---|
| | | 166 | 219 | 100 | 5045 | 75 | 84 | 1564 | 317 |
| 1 | 31446 | 8.76E-10 | 1.46E-09 | 1.83E-10 | 0 | 3.78E-09 | 3.79E-10 | 5.59E-09 | 2.30E-08 |
| 2 | 31881 | 6.59E-10 | 3.26E-08 | 2.20E-09 | 0 | 8.19E-08 | 3.65E-08 | 1.05E-09 | 4.40E-07 |
| 3 | 31866 | 6.64E-09 | 1.39E-08 | 3.84E-10 | 0 | 9.07E-08 | 3.16E-08 | 1.68E-06 | 1.06E-08 |
| 4 | 31874 | 2.40E-09 | 4.88E-07 | 0 | 5.24E-09 | 1.13E-08 | 4.12E-08 | 2.45E-09 | 3.18E-08 |
| 5 | 31549 | 0 | 0 | 1.21E-08 | 4.68E-08 | 2.55E-10 | 1.92E-09 | 3.57E-09 | 1.16E-07 |
| 6 | 31878 | 4.95E-07 | 6.70E-08 | 1.29E-07 | 0 | 7.98E-09 | 6.24E-08 | 0 | 1.27E-08 |
| 7 | 531281 | 4.38E-09 | 0 | 0 | 6.03E-09 | 6.30E-09 | 8.64E-09 | 6.32E-09 | 3.00E-08 |
| 8 | 31877 | 4.31E-10 | 1.98E-08 | 0 | 1.27E-06 | 2.26E-09 | 0 | 5.71E-09 | 4.16E-06 |
| 9 | 530373 | 0 | 1.95E-10 | 0 | 1.03E-09 | 5.36E-06 | 1.73E-06 | 6.19E-09 | 6.78E-09 |
| 10 | 531155 | 1.04E-10 | 0 | 0 | 2.06E-10 | 6.59E-09 | 5.60E-09 | 2.90E-07 | 6.04E-08 |
| 11 | 31868 | 1.01E-06 | 4.41E-09 | 2.31E-09 | 8.17E-09 | 3.01E-08 | 8.16E-09 | 2.62E-05 | 4.45E-07 |
| 12 | 531361 | 5.88E-09 | 4.01E-10 | 5.35E-09 | 0 | 0 | 0 | 5.94E-06 | 7.43E-09 |
| 13 | 531775 | 3.08E-08 | 3.43E-07 | 0 | 2.41E-09 | 0 | 3.02E-08 | 4.37E-08 | 3.53E-08 |
| 14 | 31865 | 6.51E-09 | 0 | 0 | 2.93E-07 | 1.57E-08 | 1.18E-08 | 1.69E-08 | 2.72E-08 |
| 15 | 31223 | 5.75E-08 | 9.01E-09 | 1.43E-07 | 4.10E-10 | 2.09E-09 | 2.09E-07 | 2.07E-08 | 7.67E-09 |
| 16 | 531469 | 1.62E-08 | 2.03E-09 | 0 | 5.96E-09 | 7.31E-08 | 1.92E-08 | 3.45E-08 | 3.08E-08 |
| 17 | 31876 | 1.03E-05 | 1.07E-08 | 0 | 0 | 7.33E-09 | 0 | 8.15E-08 | 2.26E-06 |
| 18 | 531416 | 6.36E-07 | 8.23E-10 | 0 | 1.80E-09 | 5.87E-08 | 6.25E-08 | 7.67E-08 | 1.10E-06 |
| 19 | 31872 | 7.96E-09 | 1.09E-09 | 0 | 1.53E-09 | 0 | 2.60E-08 | 2.21E-07 | 5.11E-09 |
| 20 | 31276 | 2.48E-08 | 0 | 5.47E-07 | 9.37E-11 | 0 | 0 | 2.29E-07 | 1.38E-08 |
| 21 | 531333 | 2.10E-08 | 0 | 0 | 1.17E-06 | 0 | 0 | 1.25E-07 | 1.43E-08 |
| 22 | 531382 | 3.82E-10 | 1.61E-08 | 0 | 9.65E-10 | 0 | 3.49E-10 | 0 | 3.75E-08 |
| 23 | 530890 | 5.16E-10 | 5.35E-09 | 5.43E-10 | 1.92E-10 | 0 | 0 | 6.17E-11 | 6.91E-07 |
| 24 | 31237 | 0 | 0 | 2.36E-08 | 0 | 0 | 1.50E-08 | 0 | 8.43E-09 |
| 25 | 31004 | 0 | 5.58E-09 | 0 | 1.78E-10 | 4.00E-09 | 2.04E-09 | 0 | 3.28E-08 |
| 26 | 531277 | 1.82E-10 | 6.93E-07 | 4.05E-09 | 8.23E-09 | 0 | 2.80E-09 | 0 | 4.19E-06 |
| 27 | 31871 | 0 | 0 | 1.05E-08 | 0 | 0 | 2.01E-09 | 0 | 3.95E-09 |
| 28 | 31188 | 0 | 1.49E-08 | 5.21E-07 | 8.76E-10 | 0 | 4.50E-09 | 0 | 1.99E-08 |
| 29 | 31875 | 1.15E-09 | 3.84E-09 | 0 | 1.02E-06 | 9.90E-08 | 1.13E-07 | 0 | 1.82E-07 |
| 30 | 31884 | 0 | 1.38E-09 | 1.81E-08 | 5.92E-09 | 0 | 0 | 8.93E-10 | 6.67E-09 |
| 31 | 31285 | 0 | 1.54E-09 | 3.09E-08 | 1.20E-06 | 0 | 0 | 0 | 1.18E-09 |
| 32 | 31489 | 3.22E-10 | 0 | 2.00E-06 | 4.04E-06 | 1.02E-08 | 4.46E-07 | 0 | 2.35E-08 |
| 33 | 31685 | 0 | 7.77E-10 | 3.58E-08 | 5.37E-09 | 0 | 0 | 0 | 1.07E-09 |
| 34 | 531248 | 7.31E-10 | 2.04E-10 | 0.000109 | 3.96E-09 | 5.12E-08 | 1.50E-07 | 0 | 6.12E-07 |
| 35 | 31870 | 0 | 0 | 1.34E-07 | 2.19E-08 | 1.26E-08 | 1.12E-08 | 4.60E-10 | 9.16E-11 |
| 36 | 531128 | 4.90E-10 | 1.78E-06 | 2.66E-07 | 8.40E-09 | 1.40E-09 | 3.65E-09 | 0 | 6.15E-09 |
| 37 | 31873 | 3.04E-09 | 3.69E-08 | 0 | 0 | 1.76E-07 | 1.77E-07 | 0 | 8.26E-07 |
| 38 | 31880 | 0 | 6.88E-08 | 0 | 0 | 5.68E-08 | 4.19E-08 | 0 | 4.58E-06 |
| 39 | 531766 | 6.19E-10 | 6.55E-06 | 2.00E-08 | 5.76E-07 | 3.27E-10 | 0 | 0 | 1.38E-05 |
| 40 | 31883 | 4.45E-08 | 1.94E-07 | 1.05E-05 | 2.46E-08 | 1.95E-07 | 2.09E-07 | 0 | 2.01E-06 |
| 41 | 531352 | 3.49E-11 | 2.64E-09 | 2.84E-09 | 0 | 0 | 0 | 5.66E-09 | 2.27E-08 |
| 42 | 31766 | 0 | 0 | 0 | 0 | 0 | 0 | 0 | 0 |
| 43 | 31537 | 0 | 0 | 7.30E-09 | 0 | 0 | 9.21E-09 | 0 | 0 |
| 44 | 31600 | 2.87E-08 | 0 | 0 | 0 | 0 | 0 | 0 | 0 |
| 45 | 31428 | 0 | 0 | 9.30E-09 | 6.89E-07 | 1.09E-10 | 0 | 0 | 0 |
| 46 | 530167 | 4.00E-09 | 1.31E-09 | 5.04E-09 | 0 | 0 | 0 | 1.37E-08 | 9.13E-09 |
| 47 | 530315 | 0 | 8.68E-10 | 0 | 0 | 0 | 0 | 0 | 6.18E-09 |
| 48 | 530050 | 2.96E-10 | 0 | 0 | 0 | 0 | 0 | 1.06E-09 | 2.83E-09 |
| 49 | 31160 | 0 | 0 | 0 | 0 | 0 | 0 | 0 | 0 |
| 50 | 530177 | 0 | 0 | 0 | 0 | 0 | 0 | 0 | 0 |
| 51 | 31700 | 1.98E-10 | 0 | 0 | 0 | 2.29E-10 | 1.97E-09 | 0 | 5.58E-08 |
| 52 | 31714 | 0 | 0 | 0 | 0 | 0 | 5.69E-09 | 0 | 0 |
| 53 | 31219 | 0 | 0 | 0 | 2.74E-08 | 0 | 0 | 0 | 2.05E-10 |
| 54 | 31557 | 0 | 0 | 0 | 0 | 0 | 0 | 0 | 0 |
| 55 | 530154 | 0 | 0 | 0 | 0 | 0 | 1.18E-08 | 0 | 5.25E-10 |
| 56 | 530444 | 0 | 0 | 2.68E-09 | 0 | 0 | 1.75E-09 | 0 | 1.28E-09 |
| 57 | 31021 | 0 | 0 | 0 | 0 | 0 | 0 | 0 | 0 |
| 58 | 530074 | 0 | 0 | 0 | 0 | 0 | 0 | 0 | 0 |
| 59 | 530227 | 0 | 0 | 0 | 0 | 0 | 0 | 0 | 0 |
| 60 | 530394 | 0 | 7.62E-10 | 0 | 4.28E-07 | 0 | 0 | 0 | 0 |
| 61 | 530295 | 0 | 0 | 6.27E-10 | 0 | 0 | 0 | 0 | 2.03E-07 |
| 62 | 530368 | 0 | 0 | 0 | 0 | 0 | 0 | 0 | 0 |
| 63 | 31300 | 0 | 1.81E-11 | 0 | 0 | 0 | 0 | 0 | 2.77E-09 |
| 64 | 31452 | 0 | 0 | 0 | 0 | 0 | 0 | 0 | 0 |
| 65 | 31723 | 0 | 1.86E-08 | 0 | 0 | 0 | 1.35E-09 | 0 | 0 |
| 66 | 531424 | 0 | 0 | 0 | 0 | 2.73E-09 | 0 | 0 | 1.22E-07 |
| 67 | 31009 | 0 | 0 | 0 | 0 | 0 | 0 | 0 | 0 |
| 68 | 530251 | 0 | 0 | 0 | 0 | 0 | 0 | 0 | 0 |
| 69 | 31416 | 0 | 0 | 0 | 0 | 0 | 0 | 0 | 0 |
| 70 | 530119 | 0 | 7.31E-08 | 0 | 0 | 0 | 0 | 0 | 9.56E-10 |
| 71 | 31749 | 0 | 0 | 0 | 0 | 0 | 0 | 0 | 2.00E-09 |
| 72 | 530364 | 0 | 0 | 0 | 0 | 0 | 0 | 0 | 9.83E-10 |
| 73 | 31328 | 0 | 0 | 0 | 0 | 8.27E-11 | 0 | 0 | 9.12E-07 |
| 74 | 530163 | 0 | 0 | 0 | 0 | 0 | 0 | 0 | 0 |
| 75 | 31379 | 0 | 0 | 2.84E-09 | 0 | 3.54E-10 | 5.94E-09 | 0 | 1.75E-06 |
| 76 | 31112 | 0 | 0 | 4.52E-09 | 0 | 0 | 1.20E-08 | 0 | 5.45E-08 |
| 77 | 31711 | 0 | 0 | 0 | 0 | 0 | 0 | 0 | 0 |
| 78 | 31750 | 0 | 0 | 0 | 0 | 0 | 7.99E-10 | 1.12E-08 | 4.99E-07 |
| 79 | 530451 | 0 | 0 | 2.99E-08 | 0 | 4.00E-10 | 8.52E-10 | 0 | 0 |
| 80 | 31129 | 0 | 4.58E-11 | 0 | 0 | 0 | 3.01E-09 | 0 | 0 |
| 81 | 530052 | 0 | 0 | 0 | 0 | 0 | 0 | 0 | 0 |
| 82 | 31512 | 0 | 0 | 0 | 0 | 0 | 0 | 0 | 0 |
| 83 | 31236 | 0 | 2.56E-09 | 0 | 0 | 0 | 0 | 0 | 1.48E-06 |
| 84 | 31360 | 3.08E-09 | 1.08E-10 | 0 | 0 | 0 | 0 | 0 | 8.08E-10 |
| 85 | 31343 | 0 | 0 | 0 | 0 | 0 | 0 | 0 | 5.12E-10 |
| 86 | 530215 | 0 | 3.67E-10 | 0 | 0 | 0 | 0 | 0 | 0 |
| 87 | 31519 | 0 | 0 | 0 | 0 | 0 | 6.02E-10 | 0 | 7.03E-08 |
| 88 | 31450 | 0 | 0 | 0 | 0 | 0 | 0 | 0 | 1.47E-05 |
| 89 | 531414 | 0 | 0 | 0 | 0 | 0 | 0 | 0 | 5.08E-07 |
| 90 | 31071 | 0 | 0 | 0 | 0 | 0 | 0 | 0 | 2.25E-10 |
| 91 | 530331 | 0 | 0 | 0 | 0 | 0 | 0 | 0 | 7.25E-09 |
| 92 | 530258 | 0 | 0 | 0 | 0 | 0 | 3.09E-09 | 0 | 2.48E-07 |
| 93 | 31333 | 0 | 0 | 0 | 0 | 0 | 0 | 0 | 0 |
| 94 | 31232 | 0 | 0 | 0 | 0 | 0 | 0 | 0 | 0 |
| 95 | 530055 | 0 | 0 | 0 | 0 | 0 | 0 | 0 | 8.87E-10 |
| 96 | 31267 | 0 | 0 | 0 | 0 | 0 | 0 | 0 | 5.32E-08 |

**Table 5: Prediction results of 15 MLGs in 156 samples**

| **samples** | **Sample ID** | **State** | **Probability of carcinoma** | **set** |
|---|---|---|---|---|
| | 31766 | controls | 0.0054 | train |
| | 31537 | controls | 0.3462 | train |
| | 31600 | controls | 0.1117 | train |
| | 31428 | controls | 0.4737 | train |
| | 530167 | controls | 0.6935 | train |
| | 530315 | controls | 0.3333 | train |
| training set (96 samples) | 530050 | controls | 0.4316 | train |
| | 31160 | controls | 0.0183 | train |
| | 530177 | controls | 0.0000 | train |
| | 31700 | controls | 0.2552 | train |
| | 31714 | controls | 0.2000 | train |
| | 31219 | controls | 0.2599 | train |
| | 31557 | controls | 0.0000 | train |
| | 530154 | controls | 0.1211 | train |
| | 530444 | controls | 0.3535 | train |
| | 31021 | controls | 0.0000 | train |
| | 530074 | controls | 0.0000 | train |
| | 530227 | controls | 0.0000 | train |
| | 530394 | controls | 0.2500 | train |
| | 530295 | controls | 0.1307 | train |
| | 530368 | controls | 0.0123 | train |
| | 31300 | controls | 0.0000 | train |
| | 31452 | controls | 0.0000 | train |
| | 31723 | controls | 0.2102 | train |
| | 531424 | controls | 0.2034 | train |
| | 31009 | controls | 0.0000 | train |
| | 530251 | controls | 0.0000 | train |
| | 31416 | controls | 0.0000 | train |
| | 530119 | controls | 0.2022 | train |
| | 31749 | controls | 0.1514 | train |
| | 530364 | controls | 0.0378 | train |
| | 31328 | controls | 0.0578 | train |
| | 530163 | controls | 0.0000 | train |
| | 31379 | controls | 0.0778 | train |
| | 31112 | controls | 0.0824 | train |
| | 31750 | controls | 0.1568 | train |
| | 530451 | controls | 0.3918 | train |
| | 31129 | controls | 0.0511 | train |
| | 530052 | controls | 0.0000 | train |
| | 31512 | controls | 0.0272 | train |
| | 31236 | controls | 0.1353 | train |
| | 31360 | controls | 0.1530 | train |
| | 31343 | controls | 0.0000 | train |
| | 530215 | controls | 0.0211 | train |
| | 31519 | controls | 0.1852 | train |
| | 31450 | controls | 0.0734 | train |
| | 531414 | controls | 0.0000 | train |
| | 31071 | controls | 0.0000 | train |
| | 530331 | controls | 0.0244 | train |
| | 530258 | controls | 0.0594 | train |
| | 31333 | controls | 0.0000 | train |
| | 31232 | controls | 0.0000 | train |
| | 530055 | controls | 0.0052 | train |
| | 31267 | controls | 0.0000 | train |
| | 31711 | controls | 0.0000 | train |
| | 31446 | carcinoma | 0.6963 | train |
| | 31881 | carcinoma | 0.8757 | train |
| | 31866 | carcinoma | 0.9789 | train |
| | 31874 | carcinoma | 0.9244 | train |
| | 31549 | carcinoma | 0.8722 | train |
| | 31878 | carcinoma | 0.9162 | train |
| | 531281 | carcinoma | 0.8226 | train |
| | 31877 | carcinoma | 0.8514 | train |
| | 530373 | carcinoma | 0.6813 | train |
| | 531155 | carcinoma | 0.9144 | train |
| | 31868 | carcinoma | 0.9946 | train |
| | 531361 | carcinoma | 0.7990 | train |
| | 531775 | carcinoma | 0.9492 | train |
| | 31865 | carcinoma | 0.9694 | train |
| | 31223 | carcinoma | 0.8619 | train |
| | 531469 | carcinoma | 0.9944 | train |
| | 31876 | carcinoma | 0.9211 | train |
| | 531416 | carcinoma | 0.9585 | train |
| | 31872 | carcinoma | 0.9063 | train |
| | 31276 | carcinoma | 0.8421 | train |
| | 531333 | carcinoma | 0.7713 | train |
| | 531382 | carcinoma | 0.4703 | train |
| | 530890 | carcinoma | 0.5179 | train |
| | 31237 | carcinoma | 0.4641 | train |
| | 31004 | carcinoma | 0.2961 | train |
| | 531277 | carcinoma | 0.8225 | train |
| | 31871 | carcinoma | 0.1320 | train |
| | 31188 | carcinoma | 0.8525 | train |
| | 31875 | carcinoma | 0.8128 | train |
| | 31884 | carcinoma | 0.6685 | train |
| | 31285 | carcinoma | 0.7967 | train |
| | 31489 | carcinoma | 0.9053 | train |
| | 31685 | carcinoma | 0.5916 | train |
| | 531248 | carcinoma | 0.9179 | train |
| | 31870 | carcinoma | 0.6898 | train |
| | 531128 | carcinoma | 0.9459 | train |
| | 31873 | carcinoma | 0.7644 | train |
| | 31880 | carcinoma | 0.5561 | train |
| | 531766 | carcinoma | 0.9372 | train |
| | 31883 | carcinoma | 0.9412 | train |
| | 531352 | carcinoma | 0.4737 | train |
| | 530075 | controls | 0.0540 | test |
| | 31637 | controls | 0.0320 | test |
| | 31398 | advanced adenoma | 0.7140 | test |
| | 531403 | advanced adenoma | 0.7520 | test |
| | 530168 | advanced adenoma | 0.2980 | test |
| | 530185 | advanced adenoma | 0.2220 | test |
| | 530600 | advanced adenoma | 0.7080 | test |
| | 31477 | advanced adenoma | 0.5560 | test |
| | 530403 | advanced adenoma | 0.0600 | test |
| | 530002 | advanced adenoma | 0.0860 | test |
| | 530697 | advanced adenoma | 0.2620 | test |
| | 31455 | advanced adenoma | 0.5800 | test |
| | 530756 | advanced adenoma | 0.0920 | test |
| | 31424 | advanced adenoma | 0.1300 | test |
| | 31501 | advanced adenoma | 0.3240 | test |
| | 31256 | advanced adenoma | 0.1420 | test |
| | 530297 | advanced adenoma | 0.5340 | test |
| | 530142 | advanced adenoma | 0.5360 | test |
| | 530026 | advanced adenoma | 0.4320 | test |
| | 530558 | advanced adenoma | 0.2140 | test |
| | 530054 | advanced adenoma | 0.3220 | test |
| | 530743 | advanced adenoma | 0.7300 | test |
| test set (60 samples) | 530867 | advanced adenoma | 0.2860 | test |
| | 530705 | advanced adenoma | 0.2820 | test |
| | 31337 | advanced adenoma | 0.0140 | test |
| | 31030 | advanced adenoma | 0.4040 | test |
| | 31282 | advanced adenoma | 0.0480 | test |
| | 530028 | advanced adenoma | 0.5340 | test |
| | 31449 | advanced adenoma | 0.2800 | test |
| | 31275 | advanced adenoma | 0.4340 | test |
| | 530018 | advanced adenoma | 0.0200 | test |
| | 530262 | advanced adenoma | 0.0540 | test |
| | 530039 | advanced adenoma | 0.4000 | test |
| | 530172 | advanced adenoma | 0.0180 | test |
| | 31137 | advanced adenoma | 0.0000 | test |
| | 31431 | advanced adenoma | 0.0440 | test |
| | 31233 | advanced adenoma | 0.0000 | test |
| | 530398 | advanced adenoma | 0.1640 | test |
| | 31582 | advanced adenoma | 0.1500 | test |
| | 530450 | advanced adenoma | 0.0820 | test |
| | 530623 | advanced adenoma | 0.1700 | test |
| | 530323 | advanced adenoma | 0.1180 | test |
| | 530348 | advanced adenoma | 0.0060 | test |
| | 530041 | advanced adenoma | 0.0000 | test |
| | 31705 | advanced adenoma | 0.2420 | test |
| | 530840 | advanced adenoma | 0.0080 | test |
| | 31367 | carcinoma | 0.9220 | test |
| | 31493 | carcinoma | 0.9840 | test |
| | 31879 | carcinoma | 0.4680 | test |
| | 531274 | carcinoma | 0.9480 | test |
| | 31159 | carcinoma | 0.8940 | test |
| | 532749 | controls | 0.0820 | test |
| | 532779 | controls | 0.5240 | test |
| | 532796 | controls | 0.2520 | test |
| | 532802 | controls | 0.0860 | test |
| | 532826 | controls | 0.3700 | test |
| | 532832 | advanced adenoma | 0.2500 | test |
| | 532305 | advanced adenoma | 0.4640 | test |
| | 532915 | controls | 0.4940 | test |
| | 531663 | advanced adenoma | 0.3600 | test |

**Table 6: sort of 15 MLGs importance**

| sort of importance | mlg ID | number of mlg | AUC |
|---|---|---|---|
| 1 | 5045 | | |
| 2 | 121 | 2 | 0.91751663 |
| 3 | 75 | 3 | 0.970731707 |
| 4 | 109 | 4 | 0.959645233 |
| 5 | 317 | 5 | 0.975609756 |
| 6 | 135 | 6 | 0.978713969 |
| 7 | 223 | 7 | 0.980044346 |
| 8 | 100 | 8 | 0.985365854 |
| 9 | 219 | 9 | 0.984035477 |
| 10 | 114 | 10 | 0.981818182 |
| 11 | 84 | 11 | 0.980931264 |
| 12 | 166 | 12 | 0.979157428 |
| 13 | 2985 | 13 | 0.987583149 |
| 14 | 131 | 14 | 0.986696231 |
| 15 | 1564 | 15 | 0.983370288 |

**Table 7-1: 10 most discriminant MLGs (species markers) associated with advanced adenoma (enrichment: according to MLG direction of enrichment in control (CTRL) vs. adenoma (AA), control (CTRL) vs. carcinoma (CRC), and adenoma (AA) vs. carcinoma (CRC), where + indicates enrichment in latter, - indicates enrichment in former, and 0 indicates no difference (p ≥ 0.05, Wilcoxon-rank sum test, Bonferroni correction to control for multiple testing).**

| MLG ID | p-value (Kruskal) | p.adjust (BH) | p-value (Wilcoxon) | | | Enrichment | Classifier | | Rank mean | | | Occurrence | | |
|---|---|---|---|---|---|---|---|---|---|---|---|---|---|---|
| | | | CTRL vs AA | CTRL vs CRC | AA vs CRC | | CRC | AA | CTRL | AA | CRC | CTRL | AA | CRC |
| 317 | 0.0000 | 0.0000 | 0.0016 | 0.0000 | 0.0412 | +,+,+ | YES | YES | 49 | 74 | 93 | 0.51 | 0.88 | 1.00 |
| 1340 | 0.0000 | 0.0000 | 0.2241 | 0.0001 | 0.0000 | 0,+,+ | NO | YES | 64 | 52 | 95 | 0.76 | 0.62 | 0.98 |
| 3840 | 0.0000 | 0.0000 | 0.0076 | 0.0000 | 0.0910 | -,-,0 | NO | YES | 89 | 64 | 48 | 0.80 | 0.40 | 0.20 |
| 665 | 0.0011 | 0.0017 | 0.0013 | 0.0111 | 1.0000 | +,+,0 | NO | YES | 56 | 81 | 76 | 0.20 | 0.52 | 0.46 |
| 3770 | 0.0001 | 0.0004 | 0.0002 | 0.0143 | 0.8448 | -,-,0 | NO | YES | 86 | 54 | 63 | 0.71 | 0.33 | 0.46 |
| 721 | 0.0000 | 0.0000 | 0.0003 | 0.0000 | 0.0929 | +,+,0 | NO | YES | 51 | 74 | 90 | 0.13 | 0.50 | 0.63 |
| 711 | 0.0000 | 0.0000 | 0.0022 | 0.0000 | 0.2128 | +,+,0 | NO | YES | 51 | 74 | 89 | 0.20 | 0.52 | 0.71 |
| 1738 | 0.0004 | 0.0008 | 0.0002 | 0.0905 | 0.2183 | +,0,0 | NO | YES | 56 | 85 | 71 | 0.22 | 0.62 | 0.44 |
| 4668 | 0.0000 | 0.0002 | 0.0091 | 0.0000 | 0.8270 | +,+,0 | NO | YES | 51 | 76 | 87 | 0.51 | 0.76 | 0.95 |
| 5954 | 0.0000 | 0.0001 | 0.0132 | 0.0000 | 0.2240 | +,+,0 | NO | YES | 52 | 74 | 89 | 0.44 | 0.74 | 0.88 |

**Table 7-2: 10 most discriminant MLGs (species markers) associated with advanced adenoma**

| MLG ID | Abundance mean | | | Odds ratio (95% CI) | | Gene NO. | MLG annotation | Best aligned strain (nucleotide) | Fraction of genes aligned (>65% identity) | Average identity of genes aligned (%) |
|---|---|---|---|---|---|---|---|---|---|---|
| | CTRL | AA | CRC | CTRL vs AA | CTRL vs CRC | | | | | |
| 317 | 4.13 E-07 | 3.77 E-07 | 8.77 E-07 | 1.02 (0.68-1.53) | 1.28 (0.8-2.05) | 120 | Bacteroides massiliensis | Bacteroides massiliensis | 0.9583 | 99.54 |
| 1340 | 1.07 | 7.03 | 3.00 | 1.14 | 2.56 (1.16-5.63) | 175 | mlg-1340 | Bacteroides | 0.5600 | 99.33 |
| | E-07 | E-08 | E-07 | (0.76-1.72) | | | | xylanisolvens | | |
| 3840 | 2.59 E-07 | 4.54 E-07 | 4.42 E-08 | 0.84 (0.53-1.32) | 0.21 (0.02-2) | 402 | B ifidobacterium animalis | Bifidobacterium animalis | 1.0000 | 99.41 |
| 665 | 1.01 E-07 | 5.80 E-09 | 1.68 E-07 | 69.15 (1.94-2459.39) | 81.33 (0.38-17357.41) | 293 | Paraprevotella clara | Paraprevotella clara | 0.9078 | 98.55 |
| 3770 | 8.50 E-09 | 1.89 E-07 | 2.24 E-08 | 0 (0-0.99) | 0.07 (0-1.63) | 229 | Streptococcus mutans | Streptococcus mutans | 0.9825 | 99.20 |
| 721 | 7.07 E-07 | 2.60 E-09 | 9.04 E-07 | 6.26557205259 73e+22 (0-1.85269840 015441e+58) | 4.61983727398773e+68 (390818561448.17-5.461 07543076802e+125) | 118 | mlg-721 | Unclassified | | |
| 711 | 7.60 E-07 | 1.40 E-08 | 8.72 E-07 | 4397459.31 (0-1617036381 2236702) | 4047307274.13 (8.33-196668696966666 7776) | 189 | mlg-711 | Unclassified | | |
| 1738 | 7.77 E-07 | 1.07 E-07 | 3.01 E-07 | 3.46 (1-11.92) | 1.36 (0.83-2.24) | 966 | mlg-1738 | Unclassified | | |
| 4668 | 1.25 E-06 | 3.24 E-07 | 2.19 E-06 | 2.18 (1.07-4.45) | 3.45 (0.72-16.5) | 456 | mlg-4668 | Unclassified | | |
| 5954 | 1.63 E-05 | 4.84 E-06 | 5.07 E-06 | 1.24 (0.75-2.03) | 1.01 (0.67-1.51) | 119 | mlg-5954 | Unclassified | | |

**Table 9: 10 MLGs relative abundance profiles in 97 samples (training set)**

| number NO. 1-55: CTRL; NO. 56-97: AA | Sample ID | MLG | | | | | | | | | |
|---|---|---|---|---|---|---|---|---|---|---|---|
| | | 1738 | 4668 | 3840 | 665 | 3770 | 711 | 1340 | 317 | 5954 | 721 |
| 1 | 31766 | 0 | 0 | 1.23E-07 | 6.70E-09 | 2.44E-08 | 0 | 1.10E-08 | 0 | 6.84E-07 | 0 |
| 2 | 31537 | 1.12E-08 | 2.65E-06 | 5.07E-06 | 0 | 1.70E-07 | 0 | 3.53E-08 | 0 | 0 | 0 |
| 3 | 31600 | 0 | 5.44E-09 | 1.44E-06 | 0 | 5.45E-06 | 0 | 3.97E-09 | 0 | 2.13E-10 | 0 |
| 4 | 31428 | 0 | 0 | 9.22E-10 | 0 | 0 | 0 | 5.64E-10 | 0 | 0 | 0 |
| 5 | 530167 | 0 | 2.66E-09 | 1.41E-09 | 0 | 8.32E-08 | 5.62E-09 | 1.94E-07 | 9.13E-09 | 0 | 2.39E-09 |
| 6 | 530315 | 0 | 0 | 1.73E-11 | 9.90E-09 | 3.54E-08 | 0 | 0 | 6.18E-09 | 1.27E-09 | 0 |
| 7 | 530050 | 0 | 0 | 0 | 0 | 0 | 0 | 2.62E-08 | 2.83E-09 | 0 | 0 |
| 8 | 31160 | 0 | 1.10E-08 | 0 | 1.99E-08 | 8.34E-10 | 5.39E-09 | 1.94E-08 | 0 | 0 | 0 |
| 9 | 530177 | 0 | 0 | 0 | 0 | 0 | 0 | 3.63E-09 | 0 | 0 | 0 |
| 10 | 31700 | 1.65E-07 | 3.43E-06 | 2.50E-08 | 0 | 0 | 0 | 6.77E-08 | 5.58E-08 | 0 | 0 |
| 11 | 31714 | 0 | 0 | 2.76E-09 | 0 | 1.18E-07 | 0 | 0 | 0 | 2.75E-07 | 0 |
| 12 | 31219 | 0 | 1.32E-06 | 3.99E-08 | 0 | 0 | 0 | 0 | 2.05E-10 | 0 | 0 |
| 13 | 31557 | 0 | 0 | 4.50E-07 | 0 | 0 | 0 | 2.44E-08 | 0 | 0 | 0 |
| 14 | 530154 | 0 | 0 | 1.02E-08 | 0 | 1.68E-08 | 0 | 0 | 5.25E-10 | 0 | 0 |
| 15 | 530444 | 0 | 0 | 0 | 0 | 4.28E-07 | 2.07E-08 | 6.32E-10 | 1.28E-09 | 8.93E-10 | 1.50E-09 |
| 16 | 31021 | 0 | 0 | 1.35E-09 | 0 | 1.04E-08 | 0 | 1.16E-09 | 0 | 0 | 0 |
| 17 | 530074 | 0 | 0 | 6.84E-10 | 3.78E-10 | 1.25E-06 | 0 | 0 | 0 | 0 | 0 |
| 18 | 530227 | 5.38E-07 | 0 | 0 | 0 | 9.70E-08 | 0 | 0 | 0 | 0 | 0 |
| 19 | 530394 | 1.02E-06 | 1.92E-07 | 0 | 0 | 0 | 5.88E-08 | 1.93E-06 | 0 | 0 | 1.54E-09 |
| 20 | 530295 | 0 | 1.25E-07 | 9.32E-07 | 0 | 0 | 7.94E-11 | 1.15E-08 | 2.03E-07 | 0 | 0 |
| 21 | 530368 | 0 | 0 | 3.89E-07 | 0 | 3.61E-07 | 0 | 0 | 0 | 0 | 0 |
| 22 | 31300 | 0 | 2.60E-06 | 8.30E-08 | 0 | 5.92E-09 | 0 | 2.09E-07 | 2.77E-09 | 0 | 0 |
| 23 | 31452 | 2.11E-07 | 1.19E-08 | 3.05E-08 | 0 | 2.44E-09 | 0 | 3.20E-09 | 0 | 0 | 0 |
| 24 | 31723 | 0 | 3.26E-08 | 3.58E-07 | 1.02E-08 | 2.82E-07 | 0 | 1.78E-08 | 0 | 7.15E-07 | 0 |
| 25 | 531424 | 0 | 2.49E-09 | 2.90E-11 | 0 | 4.13E-09 | 0 | 1.44E-07 | 1.22E-07 | 6.81E-11 | 0 |
| 26 | 31009 | 0 | 5.17E-08 | 7.20E-09 | 0 | 6.08E-11 | 2.74E-08 | 9.31E-09 | 0 | 0 | 1.98E-08 |
| 27 | 530251 | 0 | 0 | 2.81E-07 | 0 | 4.70E-10 | 0 | 0 | 0 | 0 | 0 |
| 28 | 31416 | 0 | 0 | 6.63E-10 | 0 | 0 | 0 | 1.47E-09 | 0 | 0 | 0 |
| 29 | 530119 | 3.98E-08 | 1.26E-10 | 1.01E-10 | 0 | 6.33E-09 | 0 | 5.49E-08 | 9.56E-10 | 0 | 0 |
| 30 | 31749 | 0 | 0 | 2.07E-09 | 0 | 2.33E-08 | 0 | 7.32E-10 | 2.00E-09 | 0 | 0 |
| 31 | 530364 | 0 | 5.23E-11 | 1.16E-09 | 0 | 0 | 0 | 2.31E-09 | 9.83E-10 | 1.03E-08 | 0 |
| 32 | 31328 | 1.92E-06 | 8.48E-10 | 1.89E-07 | 0 | 1.10E-08 | 0 | 5.55E-08 | 9.12E-07 | 2.38E-08 | 0 |
| 33 | 530163 | 0 | 1.96E-09 | 9.16E-10 | 0 | 8.78E-07 | 0 | 0 | 0 | 1.50E-08 | 0 |
| 34 | 31379 | 0 | 0 | 6.52E-07 | 8.22E-08 | 2.58E-07 | 0 | 2.34E-08 | 1.75E-06 | 0 | 0 |
| 35 | 31112 | 0 | 0 | 3.30E-10 | 4.88E-08 | 2.40E-08 | 0 | 1.19E-07 | 5.45E-08 | 0 | 0 |
| 36 | 31711 | 0 | 0 | 6.29E-06 | 0 | 2.06E-07 | 0 | 5.64E-09 | 0 | 7.28E-06 | 0 |
| 37 | 31750 | 0 | 3.11E-06 | 2.07E-08 | 0 | 1.81E-08 | 0 | 6.31E-08 | 4.99E-07 | 3.32E-08 | 0 |
| 38 | 530451 | 0 | 2.24E-06 | 4.42E-09 | 9.76E-09 | 8.80E-08 | 5.44E-07 | 0 | 0 | 1.06E-07 | 1.69E-08 |
| 39 | 31129 | 0 | 3.35E-10 | 1.39E-06 | 0 | 2.11E-08 | 3.15E-09 | 0 | 0 | 0.000252 | 0 |
| 40 | 530052 | 0 | 0 | 5.93E-08 | 0 | 0 | 0 | 2.52E-10 | 0 | 5.44E-08 | 0 |
| 41 | 31512 | 2.21E-08 | 0 | 9.43E-10 | 0 | 1.64E-08 | 1.71E-09 | 6.74E-11 | 0 | 5.07E-08 | 0 |
| 42 | 31236 | 0 | 0 | 0 | 7.57E-08 | 6.07E-09 | 0 | 1.73E-07 | 1.48E-06 | 2.84E-08 | 0 |
| 43 | 31360 | 0 | 0 | 4.66E-11 | 0 | 7.28E-10 | 0 | 1.07E-07 | 8.08E-10 | 1.27E-06 | 0 |
| 44 | 31343 | 0 | 0 | 2.48E-09 | 0 | 4.38E-07 | 0 | 0 | 5.12E-10 | 3.49E-08 | 0 |
| 45 | 530215 | 0 | 1.95E-08 | 0 | 0 | 9.43E-09 | 0 | 8.44E-09 | 0 | 3.74E-08 | 0 |
| 46 | 31519 | 1.86E-07 | 4.90E-07 | 5.28E-10 | 0 | 3.23E-08 | 9.03E-08 | 1.14E-07 | 7.03E-08 | 0 | 9.42E-08 |
| 47 | 31450 | 0 | 3.62E-09 | 0 | 0 | 0 | 0 | 1.03E-08 | 1.47E-05 | 0 | 0 |
| 48 | 531414 | 0 | 0 | 8.88E-10 | 0 | 0 | 0 | 1.95E-08 | 5.08E-07 | 0 | 0 |
| 49 | 31071 | 4.25E-09 | 1.21E-08 | 1.79E-09 | 9.59E-09 | 1.79E-08 | 0 | 0 | 2.25E-10 | 0 | 0 |
| 50 | 530331 | 1.78E-06 | 5.17E-08 | 0 | 0 | 3.77E-09 | 0 | 3.10E-08 | 7.25E-09 | 3.18E-09 | 0 |
| 51 | 530258 | 0 | 1.42E-06 | 1.09E-07 | 0 | 7.71E-09 | 0 | 8.25E-08 | 2.48E-07 | 4.13E-06 | 0 |
| 52 | 31333 | 0 | 3.68E-09 | 6.95E-06 | 0 | 1.72E-09 | 0 | 1.28E-08 | 0 | 5.85E-11 | 0 |
| 53 | 31232 | 0 | 0 | 9.64E-10 | 0 | 0 | 0 | 5.29E-08 | 0 | 0 | 0 |
| 54 | 530055 | 2.94E-09 | 0 | 0 | 0 | 0 | 1.33E-08 | 6.80E-08 | 8.87E-10 | 9.44E-10 | 7.18E-09 |
| 55 | 31267 | 0 | 1.99E-08 | 6.98E-08 | 4.45E-08 | 0 | 0 | 1.54E-07 | 5.32E-08 | 0 | 0 |
| 56 | 31398 | 2.48E-07 | 3.46E-08 | 0 | 4.71E-09 | 0 | 0 | 2.31E-09 | 5.78E-08 | 7.14E-07 | 0 |
| 57 | 531403 | 0 | 0 | 0 | 2.70E-09 | 0 | 0 | 0 | 1.35E-08 | 2.36E-08 | 0 |
| 58 | 530168 | 9.72E-07 | 3.83E-07 | 2.29E-07 | 0 | 1.81E-09 | 9.48E-08 | 0 | 7.12E-09 | 0.000544 | 9.45E-08 |
| 59 | 530185 | 2.61E-07 | 4.76E-06 | 3.83E-09 | 4.22E-07 | 0 | 3.55E-07 | 1.25E-06 | 1.37E-06 | 1.33E-07 | 1.89E-08 |
| 60 | 530600 | 8.63E-06 | 6.37E-07 | 3.38E-08 | 9.69E-08 | 0 | 1.14E-08 | 1.03E-07 | 4.93E-09 | 6.24E-10 | 1.48E-09 |
| 61 | 31477 | 1.47E-09 | 6.39E-07 | 3.74E-09 | 9.08E-09 | 0 | 0 | 3.05E-10 | 1.71E-09 | 1.55E-09 | 0 |
| 62 | 530403 | 1.63E-09 | 0 | 0 | 0 | 2.87E-10 | 0 | 9.81E-10 | 7.04E-10 | 1.39E-09 | 0 |
| 63 | 530002 | 0 | 0 | 0 | 0 | 3.94E-09 | 0 | 0 | 5.69E-09 | 1.63E-07 | 0 |
| 64 | 530697 | 6.19E-07 | 1.22E-09 | 0 | 0 | 3.83E-08 | 8.23E-08 | 2.29E-08 | 8.19E-09 | 1.34E-07 | 8.76E-09 |
| 65 | 31455 | 0 | 3.49E-07 | 0 | 0 | 0 | 1.23E-07 | 1.10E-06 | 1.25E-06 | 1.50E-06 | 1.27E-08 |
| 66 | 530756 | 0 | 0 | 8.98E-09 | 0 | 4.47E-09 | 0 | 0 | 0 | 0 | 0 |
| 67 | 31424 | 4.60E-09 | 1.03E-08 | 0 | 6.31E-09 | 0 | 0 | 0 | 0 | 0 | 0 |
| 68 | 31501 | 0 | 0 | 0 | 3.19E-08 | 7.73E-09 | 0 | 1.62E-08 | 6.41E-10 | 0 | 0 |
| 69 | 31256 | 0 | 4.76E-09 | 0 | 1.64E-08 | 0 | 0 | 0 | 6.17E-08 | 0 | 0 |
| 70 | 530297 | 0 | 2.24E-06 | 5.49E-07 | 4.05E-09 | 0 | 1.23E-07 | 4.28E-09 | 5.81E-09 | 1.25E-08 | 7.78E-09 |
| 71 | 530142 | 0 | 1.74E-09 | 0 | 4.31E-08 | 0 | 0 | 7.92E-09 | 1.33E-07 | 1.92E-08 | 0 |
| 72 | 530026 | 2.22E-08 | 2.95E-08 | 7.29E-07 | 6.84E-08 | 0 | 2.19E-08 | 1.92E-09 | 1.27E-09 | 0 | 1.37E-10 |
| 73 | 530558 | 4.67E-06 | 2.37E-06 | 0 | 0 | 0 | 0 | 2.53E-09 | 7.67E-10 | 0 | 0 |
| 74 | 530054 | 2.05E-07 | 4.02E-06 | 0 | 3.62E-09 | 0 | 1.38E-07 | 4.00E-09 | 5.49E-09 | 0 | 1.37E-07 |
| 75 | 530743 | 1.37E-06 | 2.21E-07 | 0 | 1.21E-06 | 0 | 0 | 2.54E-07 | 9.28E-09 | 0 | 0 |
| 76 | 530867 | 6.15E-07 | 4.25E-06 | 0 | 1.46E-06 | 0 | 1.50E-07 | 2.21E-07 | 7.23E-06 | 0 | 1.10E-08 |
| 77 | 530705 | 7.05E-06 | 4.75E-09 | 0 | 0 | 5.90E-08 | 1.31E-09 | 0 | 1.78E-08 | 1.49E-05 | 8.96E-10 |
| 78 | 31337 | 0 | 5.33E-09 | 2.31E-07 | 0 | 0 | 0 | 0 | 2.98E-09 | 0 | 0 |
| 79 | 31030 | 1.19E-06 | 3.45E-06 | 0 | 1.87E-07 | 9.99E-08 | 1.35E-09 | 1.78E-08 | 4.46E-09 | 1.65E-08 | 2.47E-09 |
| 80 | 31282 | 2.15E-07 | 1.28E-08 | 0 | 1.86E-07 | 0 | 8.23E-10 | 1.88E-09 | 9.17E-07 | 1.90E-08 | 2.01E-09 |
| 81 | 530028 | 7.69E-10 | 6.34E-06 | 4.31E-08 | 0 | 0 | 1.72E-09 | 1.13E-06 | 2.58E-06 | 2.17E-08 | 2.02E-09 |
| 82 | 31449 | 4.31E-08 | 4.85E-09 | 0 | 5.09E-08 | 0 | 3.01E-09 | 1.38E-09 | 1.01E-07 | 2.63E-08 | 2.29E-09 |
| 83 | 31275 | 0 | 1.52E-05 | 0 | 2.33E-08 | 0 | 3.04E-05 | 0 | 5.10E-08 | 1.30E-06 | 2.93E-05 |
| 84 | 530018 | 0 | 2.24E-10 | 0 | 0 | 1.35E-08 | 1.97E-09 | 1.53E-10 | 4.02E-10 | 9.62E-05 | 1.26E-09 |
| 85 | 530262 | 0 | 0 | 8.81E-10 | 1.23E-07 | 3.83E-09 | 0 | 1.60E-09 | 7.68E-07 | 3.09E-08 | 0 |
| 86 | 530039 | 0 | 0 | 0 | 0 | 0 | 0 | 0 | 2.23E-09 | 0 | 0 |
| 87 | 530172 | 0 | 0 | 2.20E-06 | 0 | 3.89E-09 | 0 | 0 | 0 | 4.74E-09 | 0 |
| 88 | 31137 | 0 | 0 | 8.47E-10 | 0 | 0 | 0 | 0 | 0 | 1.21E-09 | 0 |
| 89 | 31431 | 8.66E-07 | 2.36E-06 | 1.18E-08 | 0 | 1.97E-09 | 1.66E-07 | 3.05E-07 | 3.20E-07 | 3.43E-06 | 1.39E-08 |
| 90 | 31233 | 0 | 6.05E-11 | 5.75E-06 | 0 | 0 | 0 | 7.06E-10 | 0 | 1.06E-05 | 0 |
| 91 | 530398 | 3.92E-10 | 1.09E-06 | 0 | 6.78E-08 | 0 | 1.63E-09 | 3.35E-09 | 3.19E-08 | 1.71E-06 | 3.06E-09 |
| 92 | 31582 | 5.03E-07 | 3.47E-09 | 3.65E-10 | 0 | 1.14E-07 | 4.48E-08 | 0 | 1.68E-08 | 3.70E-09 | 3.49E-09 |
| 93 | 530450 | 3.03E-06 | 4.06E-07 | 1.09E-06 | 0 | 0 | 2.66E-10 | 0 | 8.70E-09 | 5.67E-10 | 0 |
| 94 | 530623 | 1.57E-06 | 2.92E-07 | 0 | 0 | 0 | 1.60E-07 | 1.89E-08 | 2.15E-06 | 9.73E-10 | 1.51E-08 |
| 95 | 530323 | 1.80E-09 | 0 | 0 | 3.08E-08 | 0 | 0 | 0 | 2.17E-09 | 6.75E-10 | 0 |
| 96 | 530348 | 2.35E-09 | 1.73E-08 | 1.94E-10 | 0 | 3.60E-09 | 0 | 0 | 7.32E-10 | 1.14E-05 | 0 |
| 97 | 530041 | 5.14E-07 | 3.45E-06 | 0 | 2.08E-07 | 0 | 5.03E-08 | 1.52E-08 | 1.78E-07 | 2.41E-08 | 4.41E-09 |

**Table 10: Prediction results of 10 MLGs in 127 samples**

| **samples** | **Sample ID** | **State** | **Probability of advanced adenoma** | **set** |
|---|---|---|---|---|
| | 31766 | controls | 0.0688 | train |
| | 31537 | controls | 0.0699 | train |
| | 31600 | controls | 0.0856 | train |
| | 31428 | controls | 0.2889 | train |
| | 530167 | controls | 0.2959 | train |
| | 530315 | controls | 0.5099 | train |
| | 530050 | controls | 0.3613 | train |
| | 31160 | controls | 0.4444 | train |
| | 530177 | controls | 0.2912 | train |
| | 31700 | controls | 0.4061 | train |
| training set | 31714 | controls | 0.0914 | train |
| (97 samples) | 31219 | controls | 0.5274 | train |
| | 31557 | controls | 0.0760 | train |
| | 530154 | controls | 0.1684 | train |
| | 530444 | controls | 0.5155 | train |
| | 31021 | controls | 0.1229 | train |
| | 530074 | controls | 0.1190 | train |
| | 530227 | controls | 0.4082 | train |
| | 530394 | controls | 0.4686 | train |
| | 530295 | controls | 0.3122 | train |
| | 530368 | controls | 0.0611 | train |
| | 31300 | controls | 0.1630 | train |
| | 31452 | controls | 0.2462 | train |
| | 31723 | controls | 0.0387 | train |
| | 531424 | controls | 0.0884 | train |
| | 31009 | controls | 0.2821 | train |
| | 530251 | controls | 0.4615 | train |
| | 31416 | controls | 0.2607 | train |
| | 530119 | controls | 0.1828 | train |
| | 31749 | controls | 0.1818 | train |
| | 530364 | controls | 0.5621 | train |
| | 31328 | controls | 0.3667 | train |
| | 530163 | controls | 0.1838 | train |
| | 31379 | controls | 0.1946 | train |
| | 31112 | controls | 0.1508 | train |
| | 31750 | controls | 0.0941 | train |
| | 530451 | controls | 0.4346 | train |
| | 31129 | controls | 0.4010 | train |
| | 530052 | controls | 0.5401 | train |
| | 31512 | controls | 0.2486 | train |
| | 31236 | controls | 0.5000 | train |
| | 31360 | controls | 0.3492 | train |
| | 31343 | controls | 0.1288 | train |
| | 530215 | controls | 0.3660 | train |
| | 31519 | controls | 0.6313 | train |
| | 31450 | controls | 0.4874 | train |
| | 531414 | controls | 0.1297 | train |
| | 31071 | controls | 0.3240 | train |
| | 530331 | controls | 0.6867 | train |
| | 530258 | controls | 0.2193 | train |
| | 31333 | controls | 0.2036 | train |
| | 31232 | controls | 0.0209 | train |
| | 530055 | controls | 0.5901 | train |
| | 31267 | controls | 0.3929 | train |
| | 31711 | controls | 0.2209 | train |
| | 31398 | advanced adenoma | 0.8505 | train |
| | 531403 | advanced adenoma | 0.8989 | train |
| | 530168 | advanced adenoma | 0.8743 | train |
| | 530185 | advanced adenoma | 0.8811 | train |
| | 530600 | advanced adenoma | 0.6477 | train |
| | 31477 | advanced adenoma | 0.6719 | train |
| | 530403 | advanced adenoma | 0.5775 | train |
| | 530002 | advanced adenoma | 0.6600 | train |
| | 530697 | advanced adenoma | 0.5615 | train |
| | 31455 | advanced adenoma | 0.6541 | train |
| | 530756 | advanced adenoma | 0.1170 | train |
| | 31424 | advanced adenoma | 0.4033 | train |
| | 31501 | advanced adenoma | 0.1404 | train |
| | 31256 | advanced adenoma | 0.7368 | train |
| | 530297 | advanced adenoma | 0.6480 | train |
| | 530142 | advanced adenoma | 0.6126 | train |
| | 530026 | advanced adenoma | 0.4890 | train |
| | 530558 | advanced adenoma | 0.4153 | train |
| | 530054 | advanced adenoma | 0.7677 | train |
| | 530743 | advanced adenoma | 0.5167 | train |
| | 530867 | advanced adenoma | 0.7956 | train |
| | 530705 | advanced adenoma | 0.6721 | train |
| | 31337 | advanced adenoma | 0.3369 | train |
| | 31030 | advanced adenoma | 0.7073 | train |
| | 31282 | advanced adenoma | 0.9665 | train |
| | 530028 | advanced adenoma | 0.5202 | train |
| | 31449 | advanced adenoma | 0.9461 | train |
| | 31275 | advanced adenoma | 0.8391 | train |
| | 530018 | advanced adenoma | 0.2874 | train |
| | 530262 | advanced adenoma | 0.4877 | train |
| | 530039 | advanced adenoma | 0.4916 | train |
| | 530172 | advanced adenoma | 0.4525 | train |
| | 31137 | advanced adenoma | 0.3476 | train |
| | 31431 | advanced adenoma | 0.7656 | train |
| | 31233 | advanced adenoma | 0.2500 | train |
| | 530398 | advanced adenoma | 0.9209 | train |
| | 31582 | advanced adenoma | 0.5062 | train |
| | 530450 | advanced adenoma | 0.6867 | train |
| | 530623 | advanced adenoma | 0.7914 | train |
| | 530323 | advanced adenoma | 0.8802 | train |
| | 530348 | advanced adenoma | 0.6344 | train |
| | 530041 | advanced adenoma | 0.9672 | train |
| | V1 | controls | 0.0187 | test |
| | V2 | controls | 0.0180 | test |
| | V3 | controls | 0.0218 | test |
| | V4 | controls | 0.0521 | test |
| | V5 | controls | 0.1012 | test |
| | V6 | controls | 0.1130 | test |
| | V7 | controls | 0.1425 | test |
| | V8 | controls | 0.1965 | test |
| | V9 | controls | 0.1997 | test |
| | V10 | controls | 0.2947 | test |
| | V11 | controls | 0.3476 | test |
| | V12 | controls | 0.5040 | test |
| | V13 | advanced adenoma | 0.4572 | test |
| | V14 | advanced adenoma | 0.4587 | test |
| | V15 | advanced adenoma | 0.4743 | test |
| | V16 | advanced adenoma | 0.4744 | test |
| | V17 | advanced adenoma | 0.4876 | test |
| | V18 | controls | 0.5011 | test |
| | V19 | controls | 0.5080 | test |
| | V20 | advanced adenoma | 0.5370 | test |
| | V21 | controls | 0.5474 | test |
| | V22 | advanced adenoma | 0.6626 | test |
| | V23 | advanced adenoma | 0.6816 | test |
| | V24 | advanced adenoma | 0.6923 | test |
| | V25 | advanced adenoma | 0.6934 | test |
| test set | V26 | advanced adenoma | 0.8108 | test |
| (30 samples) | V27 | advanced adenoma | 0.9218 | test |
| | V28 | advanced adenoma | 0.9245 | test |
| | V29 | advanced adenoma | 0.9499 | test |
| | V30 | advanced adenoma | 0.9662 | test |

**Table 11: sort of 10 MLGs importance**

| sort of importance | mlg ID | number of mlg | AUC |
|---|---|---|---|
| 1 | 317 | | |
| 2 | 3770 | 2 | 0.782251082 |
| 3 | 3840 | 3 | 0.805194805 |
| 4 | 665 | 4 | 0.773160173 |
| 5 | 721 | 5 | 0.795238095 |
| 6 | 1738 | 6 | 0.780952381 |
| 7 | 1340 | 7 | 0.895670996 |
| 8 | 5954 | 8 | 0.896536797 |
| 9 | 711 | 9 | 0.884848485 |
| 10 | 4668 | 10 | 0.873809524 |

Thus the inventors have identified and validated 15 MLGs for early and non-invasive diagnosis of colorectal carcinoma and 10 MLGs for early and non-invasive diagnosis of colorectal adenoma by a random forest model based on the associated genes markers. And the inventors have constructed a method to evaluate the risk of colorectal carcinoma and adenoma based on these associated gut microbes.

## Claims

1. A nucleic acid biomarker set for predicting or diagnosing a disease related to microbiota in a subject or determining whether a subject is at the risk of developing the disease, comprising:
(1) MLG 5045 consisting of nucleic acid with the sequence of SEQ ID NO: 3732-3918; and
(2) MLG 121 consisting of nucleic acids with the sequence of SEQ ID NO: 3919-6548; optionally, the biomarker set further comprises one or more of the following:
(3) MLG 75 consisting of nucleic acids with the sequence of SEQ ID NO: 1350-1527;
(4) MLG 109 consisting of nucleic acids with the sequence of SEQ ID NO: 6549-7235;
(5) MLG 317 consisting of nucleic acids with the sequence of SEQ ID NO: 7581-7700;
(6) MLG 135 consisting of nucleic acids with the sequence of SEQ ID NO: 2230-3731;
(7) MLG 223 consisting of nucleic acids with the sequence of SEQ ID NO: 9892-11298;
(8) MLG 100 consisting of nucleic acids with the sequence of SEQ ID NO: 9596-9891;
(9) MLG 219 consisting of nucleic acids with the sequence of SEQ ID NO: 7701-8028;
(10) MLG 114 consisting of nucleic acids with the sequence of SEQ ID NO: 1528-2089;
(11) MLG 84 consisting of nucleic acids with the sequence of SEQ ID NO: 1-165;
(12) MLG 166 consisting of nucleic acids with the sequence of SEQ ID NO: 7236-7580;
(13) MLG 2985 consisting of nucleic acids with the sequence of SEQ ID NO: 8029-9595;
(14) MLG 131 consisting of nucleic acids with the sequence of SEQ ID NO: 166-1349; and
(15) MLG 1564 consisting of nucleic acids with the sequence of SEQ ID NO: 2090-2229; preferably, the biomarker set comprises the nucleic acid biomarkers as defined in (1)-(13), wherein the disease is colorectal carcinoma.

2. The biomarker set according to claim 1, wherein the subject is a mammal, such as a primate, preferably human.

3. A method for predicting or diagnosing a disease related to microbiota in a subject or determining whether a subject is at the risk of developing the disease, comprising the following steps:
(1) determining the level or amount of each biomarker of the biomarker set according to any one of claims 1 to 2 in a fecal sample from the subject; and
(2a) calculating a probability of the disease by comparing the level or amount of each biomarker in the sample with a training dataset using a Multivariate statistical model (such as a random Forest model);
preferably, the training dataset comprises the data about the level or amount of each biomarker of a plurality of subjects having the disease and a plurality of healthy subjects;
wherein the probability of the disease greater than a cutoff indicates that the subject has or is at the risk of developing the disease; or
(2b) comparing the level or amount of each biomarker in the sample with the level or amount of the corresponding biomarker in the control; wherein the control is a plurality of healthy subjects;
wherein as compared with the control, the increase of the level or amount of the biomarkers in the sample indicates that the subject has or is at the risk of developing the disease; wherein the disease is colorectal carcinoma.

4. The method according to claim 3, wherein the training dataset comprises the data in Tables 4-1 and 4-2, and a probability greater than a cutoff of 0.5 indicates that the subject has or is at the risk of developing the disease.

5. The method according to anyone of claims 3-4, wherein the subject is a mammal, such as a primate, preferably human.

6. The method according to anyone of claims 3-5, wherein the level or amount of each biomarker is the relative abundance of each biomarker in the sample.

7. Use of a kit in the method according to claim 3, wherein said kit comprises agents that can determine the level or amount of each biomarker of the biomarker set according to anyone of claims 1-2 in a fecal sample, wherein the disease is colorectal carcinoma, wherein the agents are selected from the group consisting of:
(a) a primer set, comprising:
(a1) one or more primers which are capable of specifically amplifying MLG 5045 consisting of SEQ ID NO: 3732-3918; and
(a2) one or more primers which are capable of specifically amplifying MLG 121 consisting of SEQ ID NO: 3919-6548;
optionally, the primer set further comprises one or more of the following primers:
(a3) one or more primers which are capable of specifically amplifying MLG 75 consisting of SEQ ID NO: 1350-1527;
(a4) one or more primers which are capable of specifically amplifying MLG 109 consisting of SEQ ID NO: 6549-7235;
(a5) one or more primers which are capable of specifically amplifying MLG 317 consisting of SEQ ID NO: 7581-7700;
(a6) one or more primers which are capable of specifically amplifying MLG 135 consisting of SEQ ID NO: 2230-3731;
(a7) one or more primers which are capable of specifically amplifying MLG 223 consisting of SEQ ID NO: 9892-11298;
(a8) one or more primers which are capable of specifically amplifying MLG 100 consisting of SEQ ID NO: 9596-9891;
(a9) one or more primers which are capable of specifically amplifying MLG 219 consisting of SEQ ID NO: 7701-8028;
(a10) one or more primers which are capable of specifically amplifying MLG 114 consisting of SEQ ID NO: 1528-2089;
(a11) one or more primers which are capable of specifically amplifying MLG 84 consisting of SEQ ID NO: 1-165;
(a12) one or more primers which are capable of specifically amplifying MLG 166 consisting of SEQ ID NO: 7236-7580;
(a13) one or more primers which are capable of specifically amplifying MLG 2985 consisting of SEQ ID NO: 8029-9595;
(a14) one or more primers which are capable of specifically amplifying MLG 131 consisting of SEQ ID NO: 166-1349; and
(a15) one or more primers which are capable of specifically amplifying MLG 1564 consisting of SEQ ID NO: 2090-2229;
preferably, the primer set comprises the primers as defined in (a1)-(a13);
(b) a probe set, comprising:
(b1) one or more probes which are capable of specifically hybridizing with MLG 5045 consisting of SEQ ID NO: 3732-3918; and
(b2) one or more probes which are capable of specifically hybridizing with MLG 121 consisting of SEQ ID NO: 3919-6548;
optionally, the probe set further comprises one or more of the following probes:
(b3) one or more probes which are capable of specifically hybridizing with MLG 75 consisting of SEQ ID NO: 1350-1527;
(b4) one or more probes which are capable of specifically hybridizing with MLG 109 consisting of SEQ ID NO: 6549-7235;
(b5) one or more probes which are capable of specifically hybridizing with MLG 317 consisting of SEQ ID NO: 7581-7700;
(b6) one or more probes which are capable of specifically hybridizing with MLG 135 consisting of SEQ ID NO: 2230-3731;
(b7) one or more probes which are capable of specifically hybridizing with MLG 223 consisting of SEQ ID NO: 9892-11298;
(b8) one or more probes which are capable of specifically hybridizing with MLG 100 consisting of SEQ ID NO: 9596-9891;
(b9) one or more probes which are capable of specifically hybridizing with MLG 219 consisting of SEQ ID NO: 7701-8028;
(b10) one or more probes which are capable of specifically hybridizing with MLG 114 consisting of SEQ ID NO: 1528-2089;
(b11) one or more probes which are capable of specifically hybridizing with MLG 84 consisting of SEQ ID NO: 1-165;
(b12) one or more probes which are capable of specifically hybridizing with MLG 166 consisting of SEQ ID NO: 7236-7580;
(b13) one or more probes which are capable of specifically hybridizing with MLG 2985 consisting of SEQ ID NO: 8029-9595;
(b14) one or more probes which are capable of specifically hybridizing with MLG 131 consisting of SEQ ID NO: 166-1349; and
(b15) one or more probes which are capable of specifically hybridizing with MLG 1564 consisting of SEQ ID NO: 2090-2229;
preferably, the probe set comprises the probes as defined in (b1)-(b13);(c) a microarray comprising the primer set of (a) and/or the probe set of (b); and
(d) any combination of (a)-(c).

8. The use of the kit according to claim 7, wherein the kit further comprises addition agents, such as an agent for treating the sample (e.g. sterile water), an agent for carrying out PCR amplification (e.g. polymerase, dNTP, and amplification buffer), and an agent for carrying out hybridization (such as labeling buffer, hybridization buffer, and washing buffer).

## Patentansprüche

1. Nukleinsäure-Biomarker-Set zum Vorhersagen oder Diagnostizieren einer Krankheit in Verbindung mit Mikrobiota bei einem Subjekt, oder zum Bestimmen, ob bei einem Subjekt das Risiko besteht, die Krankheit zu entwickeln, umfassend:
(1) MLG 5045, bestehend aus Nukleinsäure mit der Sequenz von SEQ ID NO: 3732-3918; und
(2) MLG 121, bestehend aus Nukleinsäuren mit der Sequenz von SEQ ID NO: 3919-6548; optional umfasst das Biomarker-Set ferner eines oder mehrere der Folgenden:
(3) MLG 75, bestehend aus Nukleinsäuren mit der Sequenz von SEQ ID NO: 1350-1527;
(4) MLG 109, bestehend aus Nukleinsäuren mit der Sequenz von SEQ ID NO: 6549-7235;
(5) MLG 317, bestehend aus Nukleinsäuren mit der Sequenz von SEQ ID NO: 7581-7700;
(6) MLG 135, bestehend aus Nukleinsäuren mit der Sequenz von SEQ ID NO: 2230-3731;
(7) MLG 223, bestehend aus Nukleinsäuren mit der Sequenz von SEQ ID NO: 9892-11298;
(8) MLG 100 bestehend aus Nukleinsäuren mit der Sequenz von SEQ ID NO: 9596-9891;
(9) MLG 219, bestehend aus Nukleinsäuren mit der Sequenz von SEQ ID NO: 7701-8028;
(10) MLG 114, bestehend aus Nukleinsäuren mit der Sequenz von SEQ ID NO: 1528-2089;
(11) MLG 84, bestehend aus Nukleinsäuren mit der Sequenz von SEQ ID NO: 1-165;
(12) MLG 166, bestehend aus Nukleinsäuren mit der Sequenz von SEQ ID NO: 7236-7580;
(13) MLG 2985, bestehend aus Nukleinsäuren mit der Sequenz von SEQ ID NO: 8029-9595;
(14) MLG 131, bestehend aus Nukleinsäuren mit der Sequenz von SEQ ID NO: 166-1349; und
(15) MLG 1564, bestehend aus Nukleinsäuren mit der Sequenz von SEQ ID NO: 2090-2229; vorzugsweise umfasst das Biomarker-Set die Nukleinsäure-Biomarker, wie sie in (1)-(13) definiert sind, wobei die Krankheit kolorektales Karzinom ist.

2. Biomarker-Set nach Anspruch 1, wobei das Subjekt ein Säugetier ist, wie beispielsweise ein Primat, vorzugsweise ein Mensch.

3. Verfahren zum Vorhersagen oder Diagnostizieren einer Krankheit in Verbindung mit Mikrobiota bei einem Subjekt, oder zum Bestimmen, ob bei einem Subjekt das Risiko besteht, die Krankheit zu entwickeln, umfassend die folgenden Schritte:
(1) Bestimmen des Pegels oder der Menge von jedem Biomarker des Biomarker-Sets gemäß einem der Ansprüche 1 bis 2 in einer Stuhlprobe von dem Subjekt; und
(2a) Berechnen einer Wahrscheinlichkeit für die Krankheit durch Vergleichen des Pegels oder der Menge von jedem Biomarker in der Probe mit einem Trainingsdatensatz unter Verwendung eines multivariaten statistischen Modells (wie z. B. eines Random-Forest-Modells);
vorzugsweise umfasst der Trainingsdatensatz die Daten über den Pegel oder die Menge von jedem Biomarker einer Vielzahl von Subjekten, die die Krankheit aufweisen, und einer Vielzahl von gesunden Subjekten;
wobei die Wahrscheinlichkeit der Krankheit, die größer als ein Grenzwert ist, angibt, dass das Subjekt die Krankheit hat oder bei dem das Risiko besteht, die Krankheit zu entwickeln; oder
(2b) Vergleichen des Pegels oder der Menge von jedem Biomarker in der Probe mit dem Pegel oder der Menge des entsprechenden Biomarkers in der Kontrolle; wobei die Kontrolle eine Vielzahl von gesunden Subjekten ist;
wobei im Vergleich zur Kontrolle der Anstieg des Pegels oder der Menge der Biomarker in der Probe angibt, dass das Subjekt die Krankheit hat oder bei dem das Risiko besteht, die Krankheit zu entwickeln;
wobei die Krankheit kolorektales Karzinom ist.

4. Verfahren nach Anspruch 3, wobei der Trainingsdatensatz die Daten in Tabellen 4-1 und 4-2 umfasst und eine Wahrscheinlichkeit, die größer als ein Grenzwert von 0,5 ist, angibt, dass das Subjekt die Krankheit hat oder bei dem das Risiko besteht, die Krankheit zu entwickeln.

5. Verfahren nach einem der Ansprüche 3 bis 4, wobei das Subjekt ein Säugetier ist, wie beispielsweise ein Primat, vorzugsweise ein Mensch.

6. Verfahren nach einem der Ansprüche 3 bis 5, wobei der Pegel oder die Menge von jedem Biomarker die relative Häufigkeit von jedem Biomarker in der Probe ist.

7. Verwendung eines Kits in dem Verfahren nach Anspruch 3, wobei das Kit Agenzien umfasst, die den Pegel oder die Menge von jedem Biomarkers des Biomarker-Sets nach einem der Ansprüche 1 bis 2 in einer Stuhlprobe bestimmen können, wobei die Krankheit kolorektales Karzinom ist, wobei die Agenzien ausgewählt sind aus der Gruppe bestehend aus:
(a) einem Primer-Set, umfassend:
(a1) einen oder mehrere Primer, die in der Lage sind, spezifisch MLG 5045 bestehend aus SEQ ID NO: 3732-3918 zu amplifizieren; und
(a2) einen oder mehrere Primer, die in der Lage sind, spezifisch MLG 121 bestehend aus SEQ ID NO: 3919-6548 zu amplifizieren; und
optional umfasst das Primer-Set ferner einen oder mehrere der folgenden Primer:
(a3) einen oder mehrere Primer, die in der Lage sind, spezifisch MLG 75 bestehend aus SEQ ID NO: 1350-1527 zu amplifizieren; und
(a4) einen oder mehrere Primer, die in der Lage sind, spezifisch MLG 109 bestehend aus SEQ ID NO: 6549-7235 zu amplifizieren; und
(a5) einen oder mehrere Primer, die in der Lage sind, spezifisch MLG 317 bestehend aus SEQ ID NO: 7581-7700 zu amplifizieren; und
(a6) einen oder mehrere Primer, die in der Lage sind, spezifisch MLG 135 bestehend aus SEQ ID NO: 2230-3731 zu amplifizieren; und
(a7) einen oder mehrere Primer, die in der Lage sind, spezifisch MLG 223 bestehend aus SEQ ID NO: 9892-11298 zu amplifizieren; und
(a8) einen oder mehrere Primer, die in der Lage sind, spezifisch MLG 100 bestehend aus SEQ ID NO: 9596-9891 zu amplifizieren; und
(a9) einen oder mehrere Primer, die in der Lage sind, spezifisch MLG 219 bestehend aus SEQ ID NO: 7701-8028 zu amplifizieren; und
(a10) einen oder mehrere Primer, die in der Lage sind, spezifisch MLG 114 bestehend aus SEQ ID NO: 1528-2089 zu amplifizieren; und
(a11) einen oder mehrere Primer, die in der Lage sind, spezifisch MLG 84 bestehend aus SEQ ID NO: 1-165 zu amplifizieren; und
(a12) einen oder mehrere Primer, die in der Lage sind, spezifisch MLG 166 bestehend aus SEQ ID NO: 7236-7580 zu amplifizieren; und
(a13) einen oder mehrere Primer, die in der Lage sind, spezifisch MLG 2985 bestehend aus SEQ ID NO: 8029-9595 zu amplifizieren; und
(a14) einen oder mehrere Primer, die in der Lage sind, spezifisch MLG 131 bestehend aus SEQ ID NO: 166-1349 zu amplifizieren; und
(a15) einen oder mehrere Primer, die in der Lage sind, spezifisch MLG 1564 bestehend aus SEQ ID NO: 2090-2229 zu amplifizieren; und
vorzugsweise umfasst das Primer-Set die Primer, wie sie in (a1)-(a13) definiert sind;
(b) ein Sonden-Set, umfassend:
(a1) eine oder mehrere Sonden, die in der Lage sind, spezifisch mit MLG 5045 bestehend aus SEQ ID NO: 3732-3918 zu hybridisieren; und
(b2) eine oder mehrere Sonden, die in der Lage sind, spezifisch mit MLG 121 bestehend aus SEQ ID NO: 3919-6548 zu hybridisieren;
optional umfasst das Sonden-Set ferner eine oder mehrere der folgenden Sonden:
(b3) eine oder mehrere Sonden, die in der Lage sind, spezifisch mit MLG 75 bestehend aus SEQ ID NO: 1350-1527 zu hybridisieren;
(b4) eine oder mehrere Sonden, die in der Lage sind, spezifisch mit MLG 109 bestehend aus SEQ ID NO: 6549-7235 zu hybridisieren;
(b5) eine oder mehrere Sonden, die in der Lage sind, spezifisch mit MLG 317 bestehend aus SEQ ID NO: 7581-7700 zu hybridisieren;
(b6) eine oder mehrere Sonden, die in der Lage sind, spezifisch mit MLG 135 bestehend aus SEQ ID NO: 2230-3731 zu hybridisieren;
(b7) eine oder mehrere Sonden, die in der Lage sind, spezifisch mit MLG 223 bestehend aus SEQ ID NO: 9892-11298 zu hybridisieren;
(b8) eine oder mehrere Sonden, die in der Lage sind, spezifisch mit MLG 100 bestehend aus SEQ ID NO: 9596-9891 zu hybridisieren;
(b9) eine oder mehrere Sonden, die in der Lage sind, spezifisch mit MLG 219 bestehend aus SEQ ID NO: 7701-8028 zu hybridisieren;
(b10) eine oder mehrere Sonden, die in der Lage sind, spezifisch mit MLG 114 bestehend aus SEQ ID NO: 1528-2089 zu hybridisieren;
(b11) eine oder mehrere Sonden, die in der Lage sind, spezifisch mit MLG 84 bestehend aus SEQ ID NO: 1-165 zu hybridisieren;
(b12) eine oder mehrere Sonden, die in der Lage sind, spezifisch mit MLG 166 bestehend aus SEQ ID NO: 7236-7580 zu hybridisieren;
(b13) eine oder mehrere Sonden, die in der Lage sind, spezifisch mit MLG 2985 bestehend aus SEQ ID NO: 8029-9595 zu hybridisieren; und
(b14) eine oder mehrere Sonden, die in der Lage sind, spezifisch mit MLG 131 bestehend aus SEQ ID NO: 166-1349 zu hybridisieren;
(b15) eine oder mehrere Sonden, die in der Lage sind, spezifisch mit MLG 1564 bestehend aus SEQ ID NO: 2090-2229 zu hybridisieren; und
vorzugsweise umfasst das Sonden-Set die Sonden, wie sie in (b1)-(b13) definiert sind;(c) ein Mikroarray, umfassend das Primer-Set von (a) und/oder das Sonden-Set von (b); und
(d) eine beliebige Kombination von (a)-(c).

8. Verwendung des Kits nach Anspruch 7, wobei das Kit ferner Additive umfasst, wie z. B. ein Mittel zum Behandeln der Probe (z. B. steriles Wasser), ein Mittel zum Durchführen von PCR-Amplifikation (z. B. Polymerase, dNTP und Amplifikationspuffer) und ein Mittel zum Durchführen von Hybridisierung (z. B. Markierungspuffer, Hybridisierungspuffer und Waschpuffer).

## Revendications

1. Ensemble biomarqueur d'acide nucléique pour prédire ou diagnostiquer une maladie liée à un microbiote sur un sujet ou pour déterminer si un sujet présente un risque de développer la maladie, comprenant :
(1) MLG 5045 constitué d'acide nucléique présentant la séquence SEQ ID NO : 3732-3918 ; et
(2) MLG 121 constitué d'acide nucléique présentant la séquence SEQ ID NO : 3919-6548 ; éventuellement, l'ensemble biomarqueur comprend en outre un ou plusieurs des éléments suivants :
(3) MLG 75 constitué d'acide nucléique présentant la séquence SEQ ID NO : 1350-1527 ;
(4) MLG 109 constitué d'acide nucléique présentant la séquence SEQ ID NO : 6549-7235 ;
(5) MLG 317 constitué d'acide nucléique présentant la séquence SEQ ID NO : 7581-7700 ;
(6) MLG 135 constitué d'acide nucléique présentant la séquence SEQ ID NO : 2230-3731 ;
(7) MLG 223 constitué d'acide nucléique présentant la séquence SEQ ID NO : 9892-11298 ;
(8) MLG 100 constitué d'acide nucléique présentant la séquence SEQ ID NO : 9596-9891 ;
(9) MLG 219 constitué d'acide nucléique présentant la séquence SEQ ID NO : 7701-8028 ;
(10) MLG 114 constitué d'acide nucléique présentant la séquence SEQ ID NO : 1528-2089 ;
(11) MLG 84 constitué d'acide nucléique présentant la séquence SEQ ID NO : 1-165 ;
(12) MLG 166 constitué d'acide nucléique présentant la séquence SEQ ID NO : 7236-7580 ;
(13) MLG 2985 constitué d'acide nucléique présentant la séquence SEQ ID NO : 8029-9595 ;
(14) MLG 131 constitué d'acide nucléique présentant la séquence SEQ ID NO : 166-1349 ; et
(15) MLG 1564 constitué d'acide nucléique présentant la séquence SEQ ID NO : 2090-2229 ; de préférence, l'ensemble biomarqueur comprend les biomarqueurs d'acide nucléique, tels que définis en (1)-(13), dans lequel la maladie est un carcinome colorectal.

2. Ensemble biomarqueur selon la revendication 1, dans lequel le sujet est un mammifère, tel qu'un primate, de préférence un être humain.

3. Procédé de prédiction ou de diagnostic d'une maladie liée à un microbiote sur un sujet ou de détermination si un sujet présente un risque de développer la maladie, comprenant les étapes suivantes :
(1) la détermination du niveau ou de la quantité de chaque biomarqueur de l'ensemble biomarqueur selon l'une quelconque des revendications 1 ou 2 dans un échantillon de selles du sujet ; et
(2a) le calcul d'une probabilité de la maladie en comparant le niveau ou la quantité de chaque biomarqueur dans l'échantillon avec un ensemble de données de formation utilisant un modèle statistique multivarié (comme un modèle de forêt d'arbres décisionnels) ;
de préférence, l'ensemble de données de formation comprend les données concernant le niveau ou la quantité de chaque biomarqueur d'une pluralité de sujets présentant la maladie et d'une pluralité de sujets sains ;
dans lequel la probabilité de la maladie supérieure à un seuil indique que le sujet souffre de la maladie ou présente un risque de la développer ; ou
(2b) la comparaison du niveau ou de la quantité de chaque biomarqueur dans l'échantillon avec le niveau ou la quantité du biomarqueur correspondant dans le contrôle ; dans lequel le contrôle est constitué d'une pluralité de sujets sains ;
dans lequel, par comparaison au contrôle, l'augmentation du niveau ou de la quantité des biomarqueurs dans l'échantillon indique que le sujet souffre de la maladie ou présente un risque de la développer ;
dans lequel la maladie est un carcinome colorectal.

4. Procédé selon la revendication 3, dans lequel l'ensemble de données de formation comprend les données figurant dans les Tableaux 4-1 et 4-2, et une probabilité supérieure à un seuil de 0,5 indique que le sujet souffre de la maladie ou présente un risque de la développer.

5. Procédé selon l'une quelconque des revendications 3 à 4, dans lequel le sujet est un mammifère, tel qu'un primate, de préférence un être humain.

6. Procédé selon l'une quelconque des revendications 3 à 5, dans lequel le niveau ou la quantité de chaque biomarqueur est l'abondance relative de chaque biomarqueur dans l'échantillon.

7. Utilisation d'un kit dans le procédé selon la revendication 3, dans laquelle ledit kit comprend des agents pouvant déterminer le niveau ou la quantité de chaque biomarqueur de l'ensemble biomarqueur selon l'une quelconque des revendications 1 à 2 dans un échantillon de selles, dans laquelle la maladie est un carcinome colorectal, dans laquelle les agents sont sélectionnés dans le groupe constitué de :
(a) un ensemble d'amorce, comprenant :
(a1) une ou plusieurs amorces capables d'amplifier spécifiquement le MLG 5045 constitué de la SEQ ID NO : 3732-3918 ; et
(a2) une ou plusieurs amorces capables d'amplifier spécifiquement le MLG 121 constitué de la SEQ ID NO : 3919-6548 ;
éventuellement, l'ensemble d'amorce comprend en outre une ou plusieurs des amorces suivantes :
(a3) une ou plusieurs amorces capables d'amplifier spécifiquement le MLG 75 constitué de la SEQ ID NO : 1350-1527 ;
(a4) une ou plusieurs amorces capables d'amplifier spécifiquement le MLG 109 constitué de la SEQ ID NO : 6549-7235 ;
(a5) une ou plusieurs amorces capables d'amplifier spécifiquement le MLG 317 constitué de la SEQ ID NO : 7581-7700 ;
(a6) une ou plusieurs amorces capables d'amplifier spécifiquement le MLG 135 constitué de la SEQ ID NO : 2230-3731 ;
(a7) une ou plusieurs amorces capables d'amplifier spécifiquement le MLG 223 constitué de la SEQ ID NO : 9892-11298 ;
(a8) une ou plusieurs amorces capables d'amplifier spécifiquement le MLG 100 constitué de la SEQ ID NO : 9596-9891 ;
(a9) une ou plusieurs amorces capables d'amplifier spécifiquement le MLG 219 constitué de la SEQ ID NO : 7701-8028 ;
(a10) une ou plusieurs amorces capables d'amplifier spécifiquement le MLG 114 constitué de la SEQ ID NO : 1528-2089 ;
(a11) une ou plusieurs amorces capables d'amplifier spécifiquement le MLG 84 constitué de la SEQ ID NO : 1-165 ;
(a12) une ou plusieurs amorces capables d'amplifier spécifiquement le MLG 166 constitué de la SEQ ID NO : 7236-7580 ;
(a13) une ou plusieurs amorces capables d'amplifier spécifiquement le MLG 2985 constitué de la SEQ ID NO : 8029-9595 ;
(a14) une ou plusieurs amorces capables d'amplifier spécifiquement le MLG 131 constitué de la SEQ ID NO : 166-1349 ; et
(a15) une ou plusieurs amorces capables d'amplifier spécifiquement le MLG 1564 constitué de la SEQ ID NO : 2090-2229 ;
de préférence, l'ensemble d'amorce comprend les amorces telles que définies en (a1)-(a13) ;
(b) un ensemble de sonde, comprenant :
(b1) une ou plusieurs sondes capables de s'hybrider spécifiquement avec le MLG 5045 constitué de la SEQ ID NO : 3732-3918 ; et
(b2) une ou plusieurs sondes capables de s'hybrider spécifiquement avec le MLG 121 constitué de la SEQ ID NO : 3919-6548 ;
éventuellement, l'ensemble de sonde comprend en outre une ou plusieurs des sondes suivantes :
(b3) une ou plusieurs sondes capables de s'hybrider spécifiquement avec le MLG 75 constitué de la SEQ ID NO : 1350-1527 ;
(b4) une ou plusieurs sondes capables de s'hybrider spécifiquement avec le MLG 109 constitué de la SEQ ID NO : 6549-7235 ;
(b5) une ou plusieurs sondes capables de s'hybrider spécifiquement avec le MLG 317 constitué de la SEQ ID NO : 7581-7700 ;
(b6) une ou plusieurs sondes capables de s'hybrider spécifiquement avec le MLG 135 constitué de la SEQ ID NO : 2230-3731 ;
(b7) une ou plusieurs sondes capables de s'hybrider spécifiquement avec le MLG 223 constitué de la SEQ ID NO : 9892-11298 ;
(b8) une ou plusieurs sondes capables de s'hybrider spécifiquement avec le MLG 100 constitué de la SEQ ID NO : 9596-9891 ;
(b9) une ou plusieurs sondes capables de s'hybrider spécifiquement avec le MLG 219 constitué de la SEQ ID NO : 7701-8028 ;
(b10) une ou plusieurs sondes capables de s'hybrider spécifiquement avec le MLG 114 constitué de la SEQ ID NO : 1528-2089 ;
(b11) une ou plusieurs sondes capables de s'hybrider spécifiquement avec le MLG 84 constitué de la SEQ ID NO : 1-165 ;
(b12) une ou plusieurs sondes capables de s'hybrider spécifiquement avec le MLG 166 constitué de la SEQ ID NO : 7236-7580 ;
(b13) une ou plusieurs sondes capables de s'hybrider spécifiquement avec le MLG 2985 constitué de la SEQ ID NO : 8029-9595 ;
(b14) une ou plusieurs sondes capables de s'hybrider spécifiquement avec le MLG 131 constitué de la SEQ ID NO : 166-1349 ; et
(b15) une ou plusieurs sondes capables de s'hybrider spécifiquement avec le MLG 1564 constitué de la SEQ ID NO : 2090-2229 ;
de préférence, l'ensemble de sonde comprend les sondes telles que définies en (b1)-(b13) ; (c) un micro-réseau comprenant l'ensemble d'amorce (a) et/ou l'ensemble de sonde de (b) ; et
(d) toute combinaison de (a)-(c).

8. Utilisation du kit selon la revendication 7, dans laquelle le kit comprend en outre des agents d'addition, comme un agent pour le traitement de l'échantillon (par exemple de l'eau stérile), un agent pour effectuer une amplification PCR (par exemple polymérase, dNTP, et tampon d'amplification), et un agent pour réaliser une hybridation (comme un tampon de marquage, un tampon d'hybridation, et un tampon de lavage).
